# EUROPEAN PATENT SPECIFICATION

(11) **EP 1 751 138 B1**
(45) Date of publication and mention of the grant of the patent: **26.12.2007**
(21) Application number: 05745439.9
(22) Date of filing: 11.05.2005
(51) Int. Cl.: C07D 401/06, C07D 409/06, A61K 31/404, A61P 25/00

(54) **INDOL-2-ONE DERIVATIVES FOR THE TREATMENT OF CENTRAL NERVOUS DISORDERS, GASTROINTESTINAL DISORDERS AND CARDIOVASCULAR DISORDERS**
INDOL-2-ONDERIVATE ZUR BEHANDLUNG VON ERKRANKUNGEN DES ZENTRALEN NERVENSYSTEMS, ERKRANKUNGEN DES MAGEN-DARM-TRAKTS UND HERZKREISLAUFERKANKUNGEN
DERIVES DE L'INDOL-2-ONE TRAITANT LES TROUBLES DU SNC AINSI QUE LES TROUBLES GASTRO-INTESTINAUX ET CARDIO-VASCULAIRES

(30) Priority: 11.05.2004 HU 0400955; 05.05.2005 HU 0500463
(43) Date of publication of application: 14.02.2007
(73) Proprietor: EGIS Gyógyszergyár Nyrt, Keresztúri út 30-38 1106 Budapest (HU)
(72) Inventor: VOLK, Balázs, H-1165 Budapest (HU); BARKÓCZY, József, H-1016 Budapest (HU); SIMIG, Gyula, H-1126 Budapest (HU); MEZEI, Tibor, H-1122 Budapest (HU); KAPILLERNÉ DEZSOFI, Rita, H-1055 Budapest (HU); GACSÁLYI, István, H-1201 Budapest (HU); PALLAGI, Katalin, H-1054 Budapest (HU); GIGLER, Gábor, H-1119 Budapest (HU); LÉVAY, György, H-2092 Budakeszi (HU); MÓRICZ, Krisztina, H-1071 Budapest (HU); LEVELEKI, Csilla, H-1029 Budapest (HU); SZIRAY, Nóra, H-1025 Budapest (HU); SZÉNÁSI, Gábor, H-2096 Uröm (HU); EGYED, András, H-1145 Budapest (HU); HÁRSING, László, Gábor, H-1071 Budapest (HU)
(74) Representative: Beszédes, Stephan G.
(86) International application number: PCT/HU2005/000051
(87) International publication number: WO 2005/108390

(56) References cited:
- EP-A- 1 081 136
- WO-A-99/62900
- WO-A-02/051833
- KAPILLER R. ET AL.: "Interpretation of substituent-induced 13C NMR chemical shifts of oxindoles" NEW J. CHEM., vol. 28, 2004, pages 1214-1220, XP002344910
- VALACCHI M. ET AL.: "A new carbanionic one-carbon ring enlargement-alkylation of lactams" SYNLETT, vol. 13, 2003, pages 2025-2028, XP002344911
- ASHIMORI ET AL.: "Catalytic asymetric synthesis of quaternary carbon centers" JOURNAL OF THE AMERICAN CHEMICAL SOCIETY, vol. 120, no. 26, 1998, pages 6477-6487, XP002344913
- ASHIMORI ET AL.: "Catalytic asymetric synthesis of quaternary carbon centers" JOURNAL OF THE AMERICAN CHEMICAL SOCIETY, vol. 120, no. 26, 1998, pages 6488-6499, XP002344912
- MCCARTHY C. ET AL: "Indole alkaloid synthesis. A stereospecific preparation of functionalized cis-hexahydrocarbazoles" J. CHEM. SOC. CHEMICAL COMMUNICATIONS, vol. 22, 1989, pages 1717-1719, XP009053941
- NOZOYE ET AL.: "Reactions of 2-hydroxytryptophol: results of strong acid and alkaline treatments of 2-hydroxytryptophol" CHEM. PHARM. BULL., vol. 31, no. 9, 1983, pages 2986-2992, XP001207086
- NOZOYE T. ET AL.: "Reactions of 2-hydroxytryptophol." CHEM. PHARM. BULL., vol. 36, no. 12, 1988, pages 4980-4985, XP001207085
- SHINICHIRO SAKAI ET AL.: "Synthesis of 3-ethyloxindole derivatives and the reactivity of the amide function" YAKUGAKU ZASSHI, vol. 95, no. 12, 1975, pages 1511-1516, XP009053881
- HELLMANN H. ET AL.: "Syntheses in the oxindole series; an anomalous course of the condensation reactions of tertiary Mannich bases" CHEMISCHE BERICHTE, vol. 84, 1951, pages 901-911, XP009053884

## Description

The invention relates to new 3,3-disubstituted indol-2-one derivatives, a process for the preparation thereof, pharmaceutical compositions containing said new indol-2-one derivatives and the use of said compounds in the preparation of a medicament for the treatment of diseases.

More particularly the present invention is concerned with new 3,3-disubstituted indol-2-one derivatives of the general Formula (I), 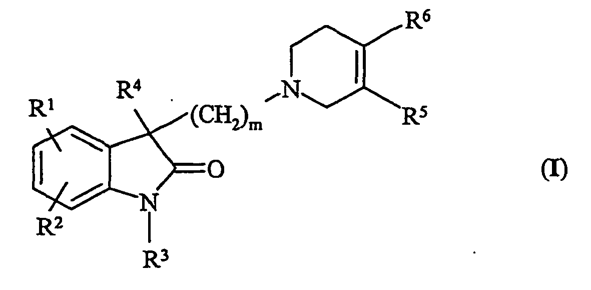 wherein
R¹ and R² independently represent hydrogen, halogen, alkyl having 1 to 7 carbon atom(s) or sulfamoyl;
R³ represents hydrogen or straight or branched chain alkyl having 1 to 7 carbon atom(s);
R⁴ stands for alkyl having 1 to 7 carbon atom(s);
R⁵ is hydrogen and R⁶ denotes phenyl optionally carrying 1 to 3 substituent(s) selected from halogen and alkyl having 1 to 7 carbon atom(s) carrying 1 to 3 halogen substituent(s), or

R⁵ and R⁶ form, together with the adjacent carbon atoms of the tetrahydropyridine ring, phenyl or a 5- or 6-membered heterocyclic ring containing a sulfur as heteroatom, which may optionally carry a halogen substituent;
m is 1, 2, 3, 4, 5 or 6,
and pharmaceutically acceptable acid addition salts thereof.

### TECHNICAL BACKGROUND OF THE INVENTION

U.S. patent No. 4,452,808 discloses 4-aminoalkyl-indol-2-one derivatives having a selective D₂ receptor activity. These compounds can be used for the treatment of hypertension. One of the compounds provided by this patent, namely 4-[2-(di-N-propylamino)ethyl]-2(3H)-indolone, is used in the clinical treatment.

European patent No. 281,309 provides indol-2-one derivatives carrying an arylpiperazinyl-alkyl substituent in position 5, which can be applied for the treatment of psychotic conditions. One of the compounds described in this patent, namely 5-[2-[4-(1,2-benzisothiazol-3-yl)-1-piperazinyl]-ethyl]-6-chloro-1,3-dihydro-*2H*-indol-2-one, exerts its activity by interaction with D₂, 5-HT_{1A} and 5-HT₂ receptors and is used in the clinical treatment.

European patent No. 376,607 discloses indol-2-one derivatives substituted in position 3 by an alkylpiperazinyl-aryl group, which exert their activity on 5-HT_{1A} receptors and are useful for the treatment of central nervous disorders.

In the international patent application WO 98/008816 indol-2-one derivatives containing a substituted alkylpiperazinyl, substituted alkyl-piperidinyl or alkyl-cyclohexyl group in position 3 are disclosed. These compounds exert anti-psychotic activity.

The acceleration of technical-social development in the XX. century constitutes a permanent compulsion of adaptation for humans, which, in adverse cases, my lead to the occurrence of adaptation disorders. Adaptation disorders constitute an important risk factor in the development of diseases of mental or psychosomatic origin, such as anxiolytic syndrome, stress disorder, depression, schizophrenia, disorders of the sense organs, gastrointestinal diseases, cardiovascular diseases or renal disorders.

For the treatment of the above clinical patterns most widespreadly pharmaceuticals exerting their activity on the benzodiazepine system (e.g. diazepam) or on central 5-HT_{1A} receptors (e.g. buspiron, ziprasidon) have been applied.

The pharmaceuticals acting on 5-HT_{1A} receptors that have been so far applied in the therapy are accompanied, however, by several drawbacks and undesired side-effects. It is a drawback that the anxiolytic effect can be achieved only after a treatment lasting for at least 10 - 14 days. Besides, after the initial administration an anxiogenic effect occurs. As to the side-effects, the occurrence of sleepiness, somnolence, vertigo, hallucination, headache, cognitive disturbances or nausea has often been observed.

### SUMMARY OF THE INVENTION

The object of the present invention is to develop pharmaceutical ingredients which are devoid of the above-specified drawbacks and undesired side-effects characteristic of the active agents binding to 5-HT_{1A} receptors and which, at the same time, can be used for the treatment of disorders of the central nervous system.

The invention is based on the recognition that the 3,3-dialkyl-substituted indol-2-one derivatives of the general Formula (I) possess a significant anxiolytic effect, but surprisingly - in contrast to the prior art compounds of similar structure - do not bind to 5-HT_{1A} receptors. As a consequence, the compounds according to the invention are devoid of the side-effects characteristic of the compounds binding to the said receptor.

### DETAILED DESCRIPTION OF THE INVENTION

According to an aspect of the present invention there are provided novel 3,3-disubstituted indol-2-on derivatives of the general Formula (I), wherein
R¹ and R² independently represent hydrogen, halogen, alkyl having 1 to 7 carbon atom(s) or sulfamoyl;
R³ represents hydrogen or straight or branched chain alkyl having 1 to 7 carbon atom(s);
R⁴ stands for alkyl having 1 to 7 carbon atom(s);
R⁵ is hydrogen and R⁶ denotes phenyl optionally carrying 1 to 3 substituent(s) selected from halogen and alkyl having 1 to 7 carbon atom(s) carrying 1 to 3 halogen substituent(s), or
R⁵ and R⁶ form, together with the adjacent carbon atoms of the tetrahydropyridine ring, a phenyl group or a 5- or 6-membered heterocyclic ring containing a sulfur as heteroatom, which may optionally carry a halogen substituent;
m is 1, 2, 3, 4, 5 or 6,
and pharmaceutically acceptable acid addition salts thereof.

The term "alkyl" used throughout this specification is intended to mean straight or branched chain saturated hydrocarbon groups having 1 to 7, preferably 1 to 4 carbon atom(s), (e.g. methyl, ethyl, 1-propyl, 2-propyl, n-butyl, isobutyl or tert. butyl group etc.)

The term "halogen" encompasses the fluorine, chlorine, bromine and iodine atoms and is preferably chlorine or bromine.

The leaving group can be an alkylsulfonyloxy or arylsulfonyloxy group, e.g. methylsulfonyloxy or p-toluenesulfonyloxy group; or a halogen atom, preferably bromine or chlorine.

The term "pharmaceutically acceptable acid addition salts" relates to non-toxic salts of the compounds of the general Formula (I) formed with pharmaceutically acceptable organic or inorganic acids. Inorganic acids suitable for salt formation are e.g. hydrogen chloride, hydrogen bromide, phosphoric, sulfuric or nitric acid. As organic acids formic, acetic, propionic, maleic, fumaric, succinic, lactic, malic, tartaric, citric, ascorbic, malonic, oxalic, mandelic, glycolic, phtalic, benzenesulfonic, p-toluene-sulfonic, naphthalic or methanesulfonic acids can be used. Furthermore, carbonates and hydro-carbonates are also considered as pharma-ceutically acceptable salts.

To a subgroup of the compounds of the general Formula (I) possessing valuable pharmaceutical properties belong the compounds wherein R¹ and R² independently represent hydrogen, straight or branched chain alkyl having 1 to 7 carbon atom(s) or halogen, R³ is hydrogen or a straight or branched chain alkyl having 1 to 7 carbon atom(s), R⁴ represents straight or branched chain alkyl having 1 to 4 carbon atom(s), R⁵ denotes hydrogen and R⁶ stands for phenyl optionally carrying a halogen or a trifluoromethyl substituent, or R⁵ and R⁶ form, together with the adjacent carbon atoms of the tetrahydropyridine ring, a 5- or 6-membered heterocyclic ring containing a sulfur atom as heteroatom and optionally carries a halogen atom, preferably chlorine, m is 3, 4 or 5, and pharmaceutically acceptable acid addition salts thereof.

To a subgroup of the compounds of the general Formula (I) possessing particularly preferable activity belong the derivatives wherein R¹, R² and R³ independently represent hydrogen or halogen, R⁴ is ethyl, R⁵ and R⁶ form, together with the adjacent carbon atoms of the dihydro-pyridine ring, a 5-membered heterocyclic ring containing sulfur as heteroatom, which optionally carries a halogen, m is 5, and pharmaceutically acceptable acid addition salts thereof.

To another subgroup of the compounds of the general Formula (I) possessing particularly preferable activity belong the derivatives wherein

R¹, R² and R³ independently represent hydrogen or halogen, R⁴ is ethyl, R⁵ stands for hydrogen, R⁶ is phenyl carrying a halogen substituent, preferably fluorine, m is 4, and pharmaceutically acceptable acid addition salts thereof.

According to a further aspect of the present invention there is provided a process for the preparation of the compounds of the general Formula (I) and pharmaceutically acceptable acid addition salts thereof, which comprises
(a) reacting a compound of the general Formula (II), 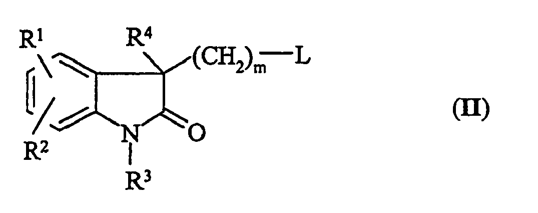 wherein R¹-R⁴ are as stated above and L is a leaving group, preferably chlorine or bromine, m is 1, 2, 3, 4, 5 or 6,, with a pyridine derivative of the general Formula (III), 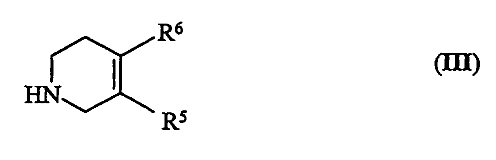 wherein R⁵ and R⁶ are as stated above, or
(b) reacting a compound of the general Formula (IV), 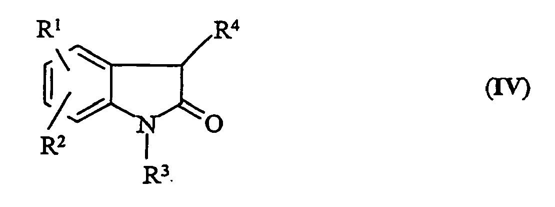 wherein R¹, R², R³ and R⁴ are as stated above, with a compound of the general Formula (V),

   L-(CH₂)ₘ-L' (V)

   wherein m is 1, 2, 3, 4, 5 or 6,, L and L' represent a leaving group, preferably chlorine or bromine, in the presence of a strong base, optionally halogenating the thus-obtained compound of the general Formula (II), wherein R² is hydrogen, and reacting the thus-obtained compound of the general Formula (II), wherein L is a leaving group, preferably chlorine or bromine, R² is hydrogen or halogen and m is 1, 2, 3, 4, 5 or 6,, with a pyridine derivative of the general Formula (III), wherein R⁵ and R⁶ are as stated above, in the presence of an acid binding agent, or
(c) reacting a compound of the general Formula (IV), wherein R¹, R², R³ and R⁴ are as stated above, with a pyridine derivative of the general Formula (VI), 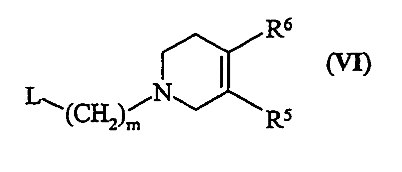
wherein L is sulfonyloxy or halogen, preferably chlorine or bromine; R⁵ and R⁶ are as stated above; m is 1, 2, 3, 4, 5 or 6, in the presence of a strong base, and optionally halogenating the thus-obtained product, wherein R² is hydrogen, or liberating the free base from a salt thereof or converting it into a pharmaceutically acceptable, organic or inorganic acid addition salts thereof.

The compounds of the general Formulae (III), (V) and (VI) are known from the literature or can be prepared by analogous methods.

When applying any one of the above process variants the desired substituents can be introduced or converted according to methods known from the literature during any one of the reaction steps. If desired, following the application of any process variant the free base corresponding to the product of the general Formula (I) can be liberated from its salt or converted into a pharmaceutically acceptable acid addition salts thereof.

When proceeding according to variant (a) the compound of the general Formula (I) can be prepared by reacting a compound of the general Formula (II) - wherein R¹-R⁴ and m are as stated above and L is a leaving group, preferably bromine or chlorine, - with a compound of the general Formula (III) - wherein R⁵ and R⁶ are as stated above - according to methods known from the literature [Houben-Weyl: Methoden der organischen Chemie, Georg Thieme Verlag, Stuttgart, 1992, 4th Edition, vol. E16d (ed.: D. Klamann); R. C. Larock: Comprehensive Organic Transformations, 2th Edition, John Wiley & Sons, New York, 1999, 789; D. A. Walsh, Y-H. Chen, J. B. Green, J. C. Nolan, J. M. Yanni J. Med. Chem. 1990, 33, 1823-1827].

During the preparation of compounds of the general Formula (II) the formation of the substituents can be performed in optional succession according to methods known from the literature. The compounds of the general Formula (II) are preferably prepared by reacting a compound of the general Formula (V) - wherein L and m are as stated above and L' is a leaving group or a group that can be converted into a leaving group - with a compound of the general Formula (IV), wherein R¹-R⁴ are as stated above [Houben-Weyl: Methoden der organischen Chemie, Georg Thieme Verlag, Stuttgart, 1977, 4th Edition, vol. V/2b; A. R. Katritzky, Ch. W. Rees: Comprehensive Heterocyclic Chemistry, First Edition, Pergamon, Oxford, 1984, vol. 4 (ed.: C. W. Bird, G. W. H. Cheeseman), 98-150 and 339-366; G. M. Karp Org. Prep. Proc. Int. 1993, 25, 481-513; B. Volk, T. Mezei, Gy. Simig Synthesis 2002, 595-597].

The compounds of the general Formula (I) can also be prepared according to process variant (c) by reacting a compound of the general Formula (IV) - wherein R¹-R⁴ are as stated above - with a compound of the general Formula (VI) - wherein R⁵, R⁶ and m are as stated above and L is a leaving group - according to methods known from the literature [R. J. Sundberg: The chemistry of indoles, Academic Press, New York, 1970, chapter VII; G. M. Karp Org. Prep. Proc. Int. 1993, 25, 481-513; A. S. Kende, J. C. Hodges Synth. Commun. 1982, 12, 1-10; W. W. Wilkerson, A. A. Kergaye, S. W. Tam J. Med. Chem. 1993, 36, 2899-2907].

During the preparation of the compounds of the general Formula (I) the formation of the substituents R¹-R⁶ can also be carried out in different successions in the last reaction step. In this case as starting material a compound of the general Formula (I) containing hydrogen in the place of the substituent to be formed. The introduction and conversion of the substituents are carried out according to methods known from the literature [Houben-Weyl: Methoden der organischen Chemie, Georg Thieme Verlag, Stuttgart, 1977, 4th Edition, IV/1a-d; vol. V/2b]. During the introduction of the substituents application or elimination of protecting groups may become necessary. Such methods are specified in T. W. Greene, Protective groups in organic synthesis, John Wiley & Sons, 1981.

The compound of the general Formula (IV), wherein R¹-R⁴ are as stated above, can be prepared by applying known methods, the formation of the substituents is carried out in optional succession according to methods known from the literature [A. R. Katritzky, Ch. W. Rees: Comprehensive Heterocyclic Chemistry, 1th Edition, Pergamon, Oxford, 1984, vol. 4 (ed.: C. W. Bird, G. W. H. Cheeseman), 98-150 and 339-366; C. Gautier, M. Aletru, Ph. Bovy WO 99/62900; B. Volk, T. Mezei, Gy. Simig Synthesis 2002, 595-597; G. M. Karp Org. Prep. Proc. Int. 1993, 25, 481-513; A. S. Kende, J. C. Hodges Synth. Commun. 1982, 12, 1-10].

The compounds of the general Formula (I) prepared by the methods according to the invention can be liberated from their salts or converted into pharmaceutically acceptable acid addition salts according to methods known from the literature.

According to a further aspect of the present invention there are provided pharmaceutical compositions comprising as active ingredient a compound of the general Formula (I) or a pharmaceutically acceptable acid addition salt thereof in admixture with one or more conventional carrier(s) or auxiliary agent(s).

The pharmaceutical compositions according to the present invention contain generally 0,1-95 % by weight, preferably 1-50 % by weight, particularly 5-30 % by weight of the active ingredient.

The pharmaceutical compositions of the present invention may be suitable for oral (e.g. powders, tablets, coated tablets, capsules, microcapsules, pills, solutions, suspensions or emulsions), parenteral (e.g. injection solutions for intravenous, intramuscular, subcutaneous or intraperitoneal use), rectal (e.g. suppositories) transdermal (e.g. plasters) or local (e.g. ointments or plasters) administration or for the application in form of implants. The solid, soft or liquid pharmaceutical compositions according to the invention may be produced by methods conventionally applied in the pharmaceutical industry.

The solid pharmaceutical compositions for oral administration containing the compounds of the general Formula (I) or pharmaceutically acceptable acid addition salts thereof may comprise fillers or carriers (such as lactose, glucose, starch, potassium phosphate, micro-crystalline cellulose), binding agents (such as gelatine, sorbite, polyvinyl pyrrolidone), disintegrants (such as croscarmelose, Na-carboxy-methyl cellulose, crospovidone), tabletting auxiliary agents (such as magnesium stearate, talc, polyethylene glycol, silicic acid, silicon dioxide) and surface-active agents (e.g. sodium lauryl sulfate).

The liquid compositions suitable for oral administration can be solutions, suspensions or emulsions. Such compositions may contain suspending agents (e.g. gelatine, carboxymethyl cellulose), emulsifiers (e.g. sorbitane mono-oleate, solvents (e.g. water, oils, glycerol, propyleneglycol, ethanol), buffering agents (e.g. acetate, phosphate, citrate buffers) and pre-servatives (e.g. methyl-4-hydroxybenzoate etc.).

Liquid pharmaceutical compositions suitable for parenteral administration are generally sterile izotonic solutions optionally containing, in addition to the solvent, buffering agents and preservatives.

Soft pharmaceutical compositions containing as active ingredient a compound of the general Formula (I) or a pharmaceutically acceptable acid addition salt thereof, such as suppositories, contain the active ingredient evenly dispersed in the basic material of the suppository (e.g. in polyethylene glycol or cocoa butter).

According to a further aspect of the present invention there is provided the use of an indol-2-one derivative of the general Formula (I) or a pharmaceutically acceptable acid addition salt thereof for the preparation of pharmaceutical compositions suitable for the treatment or prophylaxis of disorders of the central nervous system or psychosomatic disorders including anxiety syndromes, particularly generalized anxiety disorders, panic disease, compulsive disorder, social phobia, agoraphobia, phobias in connection with specific situations, post-traumatic stress disorders, post-traumatic memory disturbances, cognitive disturbances, sexual dysfunction of central nervous system origin, depression, schizophrenia, gastrointestinal diseases and cardiovascular diseases.

According to a further aspect of the present invention there is provided the use of an indol-2-one derivative of the general Formula (I) or a pharmaceutically acceptable acid addition salt thereof for the preparation of pharmaceutical compositions suitable for the treatment or prophylaxis of disorders of the central nervous system or psychosomatic disorders including anxiety syndromes, particularly generalized anxiety disorders, panic disease, compulsive disorder, social phobia, agoraphobia, phobias in connection with specific situations, post-traumatic stress disorders, memory disturbances caused by trauma, cognitive disturbances, sexual dysfunction of central nervous system origin, depression, schizophrenia, gastrointestinal diseases and cardiovascular diseases.

The pharmaceutical compositions according to the present invention can be prepared by known methods of the pharmaceutical industry. The active ingredient is admixed with pharma-ceutically acceptable solid or liquid carriers and/or auxiliary agents and the mixture is brought to galenic form. The carriers and auxiliary agents together with the methods which can be used in the pharmaceutical industry are disclosed in the literature (Remington's Pharma-ceutical Sciences, Edition 18, Mack Publishing Co., Easton, USA, 1990).

The pharmaceutical compositions according to the present invention contain generally a dosage unit. The daily dosage for human adults can be generally 0,1-1000 mg/kg body weight of a compound of the general Formula (I) or a pharmaceutically acceptable acid addition salts thereof. Said daily dose can be administered in one or more portion(s). The actual daily dose depends on several factors and is determined by the physician.

According to a further aspect of the present invention there is provided the use of the compounds of the general Formula (I) or pharmaceutically acceptable acid addition salts thereof for the treatment or prophylaxis of disorders of the central nervous system and psychosomatic disorders including anxiety syndrome, particularly generalized anxiety disorders, panic disease, compulsive disorder, social phobia, agoraphobia, phobias in connection with specific situations, stress disorders, post-traumatic memory disorders, cognitive disturbances, sexual dysfunction of central nervous system origin, depression, schizophrenia, gastrointestinal diseases and cardiovascular diseases.

It is known from the European patent specification No. 376,607 and from the technical literature (A. Dekeyne, J.M. Rivet, A. Gobert, M.J. Millan: Neuropharmacology 40(7) p. 899-910 (2001); J.S: Sprouse et al.: Neuropsychopharmacology 21(5) p.622-631 (1999); A. Newman-Tancredi et al.: Eur. J. Pharmacol. 355(2-3) pp. 245-246 (1998)) that the prior art compounds of 1,3-dihydro-2*H*-indol-2-one type selectively bind to 5-HT_{1A} receptors and thus affect the central nervous system. As a consequence, they can be used for the treatment of depression and anxiety disorders, furthermore cardiovascular, gastrointestinal and renal diseases.

The invention is based on the surprising recognition that the compounds of the general Formula (I) exert anxiolytic activity but do not bind to 5-HT_{1A} receptors. Thus, it can be expected that they are devoid of the above-listed adverse side-effects characteristic of the ingredients binding to 5-HT_{1A} receptors.

Binding of the compounds of the general Formula (I) to 5-HT_{1A} receptors was investigated according to the method of Peroutka (S.J. Peroutka: J. Neurochem. 47, p. 529 (1986)).

Receptor bindings were determined from isolated frontal cortex membrane preparation of rats by using tritiated 8-hydroxy-*N,N-*dipropyl-2-amino-tetraline (8-OH-DPAT) ligand. For the determination of non-specific binding 10 µM serotonin creatinine sulfate was used. The following conditions were applied: incubation blood volume: 250 µl, incubation temperature: 25 ° C, incubation time: 30 minutes. The reaction was terminated by the addition of 9 ml of 50 mM ice-cold TRIS-HCl buffer (pH=7,7) and quick vacuum filtration using Whatman GFIB fibreglass filtering paper. Radioactivity of the filter boards was measured with the aid of a liquid scintillation spectrometer.

IC₅₀ is the concentration whereby the difference between whole binding and non-specific binding in the presence of 10 µM serotonin creatinine sulfate is 50%. The compounds with an IC₅₀ value smaller than 100 nmol were considered effective in this test. The results are given in Table 1.

**Table 1**

| 5-HT_{1A} receptor binding experiment | |
|---|---|
| No. of Example | IC₅₀ |
| | nmole |
| 29 | >100 |
| 36 | >100 |
| 37 | >100 |
| 26 | >100 |

From the results disclosed in Table 1 it can be seen that the test compounds do not bind to 5-HT_{1A} receptors.

The anxiolytic effect of the compounds according to the invention was investigated on rats according to the so-called elevated plus-maze test (S. Pelow, P. Chopin, S.E. File, J. Briley: Neurosci. Methods 14, p. 149 (1985)).

A wooden cross elevated to 50 cm above the floor, 15 cm wide with 100 cm long arms was used for the experiments. The sides and ends of two opposite arms of the cross were equipped with 40 cm high walls, however, the arms were open to the 15 cm x 15 cm central area (closed arms). The two other opposite arms were not encircled by walls (open arms).

Male Sprague-Dawley rats weighing 200-220 g were used for the experiments. The animals were placed in the central area of the equipment 60 min after treatment and the following four parameters have been observed for the 5 min test time:
time spent in the open arms (sec),
time spent in the closed arms (sec),
number of entries into the open arms,
number of entries into the closed arms.

The effect was expressed as percent increase in either the time spent in the open arms or number of entries into the open arms. MEDs (minimal effective doses) were determined for each compound. The results are summarized in Table 5.

**Table 5**

| Elevated plus-maze in rats | |
|---|---|
| No. of Example | MED |
| | mg/kg p.o. |
| 36. | 1,0 |
| 39. | 0,01 |

From the data of the above tables it can be seen that the compounds of the general Formula (I) possess a significant anxiolytic effect.

On the basis of the above experiments the compounds according to the invention show a considerable efficacy in the treatment of disorders of the central nervous system. They may prove particularly suitable for the treatment of anxiety disorders, mixed anxiety and depression or in case of other disorders characterized by extreme stress conditions requiring tranquilliz-ation of the patient. They can also be used for the treatment of psychosomatic diseases, such as hypertension of psychic origin, gastrointestinal ulcer, colitis, asthma etc. In case of these clinical patterns it can be supposed that chronic stress, anxiety and/or unprocessed conflicts are in the background. The new compounds according to the invention surprisingly do not exert their favourable therapeutic activity via 5-HT_{1A} receptors, so it can be expected that they are devoid of the side-effects characteristic of the active ingredients acting on 5-HT_{1A} receptors.

Further details of the present invention are provided in the following examples without limiting the scope of protection to said examples.

### Example 1

### 5-Chloro-3-ethyl-1,3-dihydro-2H-indol-2-one

1.68 g (0.01 mole) of 5-chloro-oxindole is dissolved in 20 ml of ethanol, and 1.0 g of Raney-nickel is added to the solution. The reaction mixture is allowed to react in an autoclave at 110 °C for 36 hours. The catalyst is then filtered off, the solvent is evaporated and the residue is recrystallized from a mixture of hexane and ethyl acetate.
Yield: 0.86 g of white powder (44 %).
M.p.: 121-123 °C (hexane-ethyl acetate).

IR (KBr): 3156, 1701 (C=O), 782 cm⁻¹.

¹H-NMR (CDCl₃): 9.27 (br s, 1H, NH), 7.21 (1H, s, H-4), 7.19 (d, 1H, *J* = 8.8 Hz, H-6), 6.85 (d, 1H, *J* = 8.1 Hz, H-7), 3.47 (t, 1H, *J* = 5.5 Hz, H-3), 2.03 (m, 2H, CH₂), 0.92 (t, 3H, *J* = 7.0 Hz, CH₃) ppm.

¹³C-NMR (CDCl₃): 180.5, 140.4, 131.2, 127.8, 127.6, 124.5, 110.7, 47.5, 23.5, 9.9 ppm.
Elementary analysis for the Formula C₁₀H₁₀ClNO (195.65):
Calculated:C 61.39, H 5.15, N 7.16, Cl 18.12 %.
Found: C 61.16, H 5.10, N 6.93, Cl 18.11 %.

### Example 2

### 5-Bromo-3-ethyl-1,3-dihydro-2H-indol-2-one

3-ethyl oxindole (16.1 g; 0.10 mole) is dissolved in 350 ml of acetonitrile, the solution is cooled to 0 °C, and a solution of N-bromosuccinimide (17.8 g; 0.10 mole) in 150 ml of acetonitrile is dropped to it at the same temperature within 2 hours. The reaction mixture is stirred first at 0 °C for 1 hour and then at room temperature for 3 hours. The solution is evaporated, the white substance separated in crystalline form is extracted with dichloromethane and 1 M NaOH solution, and the organic phase is extracted again with alkaline water in order to remove succinimide. The organic phase is dried over sodium sulfate, filtered and evaporated. The separated white substance is recrystallized from a mixture of heptane and ethyl acetate. Yield: 15.24 g of white powder (63 %).
M.p.: 125-127 °C (heptane-ethyl acetate).
IR (KBr): 3154, 1700 (C=O), 812 cm⁻¹.

¹H-NMR (CDCl₃, TMS, 400 MHz): 8.90 (1H, s), 7.36-7.32 (2H, m), 6.81 (1H, d, *J* = 8.9 Hz), 3.43 (1H, t, *J* = 5.8 Hz), 2.03 (2H, q, *J* = 7.4 Hz), 0.92 (3H, t, *J* = 7.4 Hz) ppm.

¹³C-NMR (CDCl₃, TMS, 101 MHz): 180.3, 140.8, 131.6, 130.7, 127.2, 114.9, 111.2, 47.2, 23.4, 9.9 ppm.

| Elementary analysis for the Formula C₁₀H₁₀BrNO (240.10): | | | | |
|---|---|---|---|---|
| Calculated: | C 50.03, | H 4.20, | N 5.83, | Br 33.28 %. |
| Found: | C 50.16, | H 4.20, | N 5.85, | Br 32.70 %. |

### Preparation of ω-haloalkyl compounds (Process "A")

Into a flask rinsed with argon 2.5 M n-butyl lithium (60 ml; 0.15 mole) is measured. 200 ml of THF are added to it, and the solution is cooled in an acetone-dry ice bath to -78 °C. At this temperature a solution of the appropriate 3-alkyl oxindole (0.20 mole) in 250 ml of THF is dropped to it under stirring. The mixture is stirred for further 10 minutes, a dihaloalkane (1-bromo-4-chlorobutane, 1-bromo-3-chloropropane, 1,5-dibromopentane or 1,6-dibromohexane; 0,50 mole) is added dropwise to it, and the solution is allowed to warm up to room temperature. Then it is stirred further for 3 hours, and 20 ml of ethanol is dropped to it in order to decompose excess of butyl lithium. The solution is distilled with a rotary evaporator and the residual oil is extracted with water and ethyl acetate. The organic phase is dried over sodium sulfate. The residual oil is made crystalline by trituration with hexane. The separated off-white crystals are stirred in 200 ml of hexane in order to remove excess of dihaloalkane, filtered and washed with hexane. The product is used for the further reactions without recrystallization. Analytical samples may be obtained by recristallization from the indicated solvent.

### Example 3

### 3-(4-Chlorobutyl)-3-ethyl-1,3-dihydro-2H-indol-2-one

The title compound is prepared according to process "A" starting from 3-ethyl-1,3-dihydro-2*H*-indol-2-one and 1-bromo-4-chlorobutane.
M.p.: 104-105 °C (hexane-ethyl acetate).

IR (KBr): 3181, 2941, 1700, 1306, 755 cm⁻¹.

¹H-NMR (CDCl₃, TMS, 400 MHz): 8.57 (br s, 1H, NH), 7.21 (dt, 1H, *J* = 7.6, 1.5 Hz, H-6), 7.12 (d, 1H, *J* = 7.4 Hz, H-4), 7.06 (dt, 1H, *J* = 7.5, 1.0 Hz, H-5), 6.92 (d, 1H, *J* = 7.7 Hz, H-7), 3.39 (t, 2H, *J* = 6.7 Hz, CH₂Cl), 1.96-1.84 (m, 2H, CH₂), 1.83-1.74 (m, 2H, CH₂), 1.74-1.60 (m, 2H, CH₂), 1.24-1.18 (m, 1H), 1.08-1.03 (m, 1H), 0.64 (t, 3H, *J* = 7.4 Hz, CH₃) ppm.

¹³C-NMR (CDCl₃, TMS, 101 MHz): 182.4, 141.2, 132.3, 127.7, 123,0, 122.5, 109.6, 54.1, 44.4, 36.8, 32.7, 31.0, 21.8, 8.5 ppm.

| Elementary analysis for the Formula C₁₄H₁₈ClNO (251.76): | | | | |
|---|---|---|---|---|
| Calculated: | C 66.79, | H 7.21, | N 5.56, | Cl 14.08 %. |
| Found: | C 66.89, | H 7.16, | N 5.84, | Cl 14.19 %. |

### Example 4

### 3-(4-Chlorobutyl)-3-ethyl-5-fluoro-1,3-dihydro-2H-indol-2-one

The title compound is prepared according to process "A" starting from 3-ethyl-5-fluoro-1,3-dihydro-2*H*-indol-2-one and 1-bromo-4-chloro-butane.
M.p.: 96-97 °C (hexane-ethyl acetate).

IR (KBr): 3159, 1716, 817 cm⁻¹.

¹H-NMR (CDCl₃, TMS, 400 MHz): 8.99 (br s, 1H, NH), 6.95-6.85 (m, 3H), 3.40 (t, 2H, *J* = 6.7 Hz, CH₂Cl), 1.97-1.88 (m, 2H, CH₂), 1.83-1.75 (m, 2H, CH₂), 1.73-1.62 (m, 2H), 1.25-1.20 (m, 1H), 1.09-1.04 (m, 1H), 0.65 (t, 3H, *J* = 7.4 Hz, CH₃) ppm.

¹³C-NMR (CDCl₃, TMS, 101 MHz): 182.5, 159.3 (d, *J* = 240.7 Hz), 137.2, 134.1 (d, *J* = 7.6 Hz), 114.1 (d, *J* = 23.7 Hz), 111.9 (d, *J* = 24.4 Hz), 110.2 (d, *J* = 2.0 Hz), 54.8 (d, *J* = 2.0 Hz), 44.4, 36.8, 32.5, 31.0, 21.7, 8.4 ppm.

| Elementary analysis for the Formula C₁₄H₁₇ClFNO (269.75): | | | | |
|---|---|---|---|---|
| Calculated: | C 62.34, | H 6.35, | N 5.19, | Cl 13.14 %. |
| Found: | C 62.49, | H 6.20, | N 4.98, | Cl 13.48 %. |

### Example 5

### 3-(4-Chlorobutyl)-3-ethyl-6-fluoro-1,3-dihydro-2H-indol-2-one

The title compound is prepared according to process "A" starting from 3-ethyl-6-fluoro-1,3-dihydro-2*H*-indol-2-one and 1-bromo-4-chlorobutane.
M.p.: 95-97 °C (hexane-ethyl acetate).

IR (KBr): 3195, 1728, 1132 cm⁻¹.

¹H-NMR (CDCl₃, TMS, 400 MHz): 9.34 (br s, 1H, NH), 7.05 (dd, 1H, *J* = 8.1, 5.3 Hz, H-4), 6.75 (ddd, 1H, *J* = 9.6, 8.1, 2.4 Hz, H-5), 6.71 (dd, 1H, *J* = 8.8, 2.4 Hz, H-7), 3.44 (t, 2H, *J* = 6.7 Hz, CH₂Cl), 2.00-1.70 (m, 4H, 2 x CH₂), 1.70-1.60 (m, 2H, CH₂), 1.23-1.18 (m, 1H), 1.08-1.04 (m, 1H), 0.64 (t, 3H, *J* = 7.4 Hz, CH₃) ppm.

¹³C-NMR (CDCl₃, TMS, 101 MHz): 183.3, 162.5 (d, *J* = 244.1 Hz), 142.5 (d, *J* = 7.8 Hz), 127.5 (d, *J* = 13.0 Hz), 123.8 (d, *J* = 9.5 Hz), 108.8 (d, *J* = 22.5 Hz), 98.5 (d, *J* = 27.4 Hz), 53.8, 44.4, 36.8, 32.5, 31.0, 21.6, 8.4 ppm.

| Elementary analysis for the Formula C₁₄H₁₇ClFNO (269.75). | | | | |
|---|---|---|---|---|
| Calculated: | C 62.34, | H 6.35, | N 5.19, | Cl 13.14 %. |
| Found: | C 62.09, | H 6.22, | N 5.28, | Cl 13.43 %. |

.

### Example 6

### 3-(4-Chlorobutyl)-3-ethyl-5-methyl-1,3-dihydro-2H-indol-2-one

The title compound is prepared according to process "A" starting from 3-ethyl-5-methyl-1,3-dihydro-2*H*-indol-2-one and 1-bromo-4-chlorobutane.
M.p.: 79-80 °C (hexane).

IR (KBr): 3286, 1719 cm⁻¹.

¹H-NMR (CDCl3, TMS, 400 MHz): 8.70 (br s, 1H, NH), 7.00 (d, 1H, *J* = 7.8 Hz, H-6), 6.92 (s, 1H, H-4), 6.81 (d, 1H, *J* = 7.9 Hz, H-7), 3.39 (t, 2H, *J* = 6.8 Hz, CH₂Cl), 1.95-1.85 (m, 2H), 1.82-1.70 (m, 2H), 1.70-1.58 (m, 2H), 1.30-1.12 (m, 1H), 1.10-0.98 (m, 1H), 0.63 (t, 3H, *J* = 7.3 Hz, CH₃) ppm.

¹³C-NMR (CDCl₃, TMS, 101 MHz): 182.5, 138.8, 132.4, 131.9, 128.0, 123.7, 109.3, 54.1, 44.4, 36.9, 32.7, 31.0, 21.8, 8.4 ppm.

| Elementary analysis for the Formula C₁₅H₂₀ClNO (265.79): | | | | |
|---|---|---|---|---|
| Calculated: | C 67.79, | H 7.58, | N 5.27, | Cl 13.34 %. |
| Found: | C 67.98, | H 7.43, | N 5.11, | Cl 13.09 %. |

### Example 7

### 3-(4-Chlorobutyl)-3-ethyl-7-methyl-1,3-dihydro-2H-indol-2-one

The title compound is prepared according to process "A" starting from 3-ethyl-7-methyl-1,3-dihydro-2*H*-indol-2-one and 1-bromo-4-chlorobutane.
M.p.: 112-113 °C (hexane-ethyl acetate).

IR (KBr): 3181, 1703 (C=O), 748 cm⁻¹.

¹H-NMR (CDCl₃, TMS, 400 MHz): 0.63 (3H, t, *J* = 7.4 Hz), 1.07-1.02 (1H, m), 1.25-1.17 (1H, m), 1.70-1.60 (2H, m), 1.81-1.72 (2H, m), 1.96-1.86 (2H, m), 2.31 (3H, s), 3.36 (2H, t, *J* = 6.8 Hz), 6.94 (1H, dd, *J* = 1.7, 7.3 Hz), 6.97 (1H, t, *J* = 7.3 Hz), 7.03 (1H, dd, *J* = 1.4, 7.2 Hz), 9.4 (1H, br s) ppm.

¹³C-NMR (CDCl₃, TMS, 101 MHz): 8.5, 16.5, 21.8, 31.0, 32.7, 36.8, 44.4, 54.4, 119.1, 120.3, 122.4, 129.1, 131.9, 140.1, 183.1 ppm.

| Elementary analysis for the Formula C₁₅H₂₀ClNO (265.79): | | | | |
|---|---|---|---|---|
| Calculated: | C 67.79, | H 7.58, | N 5.27, | Cl 13.34 %. |
| Found: | C 67.56, | H 7.49, | N 5.24, | Cl 13.29 %. |

### Example 8

### 3-(3-Chloropropyl)-3-ethyl-1,3-dihydro-2H-indol-2-one

The title compound is prepared according to process "A" starting from 3-ethyl-1,3-dihydro-2*H*-indol-2-one and 1-bromo-3-chloropropane.
M.p.: 91-93 °C (hexane).

IR (KBr): 3183, 1701, 751 cm⁻¹.

¹H-NMR (CDCl₃, TMS, 400 MHz): 9.15 (br s, 1H, NH), 7.23 (dt, 1H, *J* = 7.7, 1.3 Hz, H-6), 7.14 (d, 1H, *J* = 6.8 Hz, H-4), 7.06 (dt, 1H, *J* = 7.4, 0.9 Hz, H-5), 6.95 (d, 1H, *J* = 7.7 Hz, H-7), 3.48-3.3 6 (m, 2H, CH₂Cl), 2.02-1.93 (m, 3H), 1.85-1.78 (m, 1H), 1.66-1.54 (m, 1H), 1.44-1.30 (m, 1H), 0.65 (t, 3H, *J* = 7.4 Hz, CH₃) ppm.

¹³C-NMR (CDCl₃, TMS, 101 MHz): 182.6, 141.3, 132.0, 127.9, 123.0, 122.6, 109.8, 53.7, 44.8, 34.8, 31.0, 27.5, 8.5 ppm.

| Elementary analysis for the Formula C₁₃H₁₆ClNO (237.73): | | | | |
|---|---|---|---|---|
| Calculated: | C 65.68, | H 6.78, | N 5.89, | Cl 14.91 %. |
| Found: | C 65.51, | H 6.70, | N 5.82, | Cl 14.68 %. |

### Example 9

### 3-(5-Bromopentyl)-3-ethyl-1,3-dihydro-2H-indol-2-one

The title compound is prepared according to process "A" starting from 3-ethyl-1,3-dihydro-2*H*-indol-2-one and 1,5-dibromopentane.
M.p.: 77-78 °C (hexane).

IR (KBr): 3290, 1718, 772 cm⁻¹.

¹H-NMR (CDCl₃, TMS, 400 MHz): 9.11 (br s, 1H, NH), 7.20 (dt, 1H, *J* = 7.6, 1.4 Hz, H-6), 7.11 (d, 1H, *J* = 7.3 Hz, H-4), 7.05 (dt, 1H, *J* = 7.4, 1.0 Hz, H-5), 6.94 (d, 1H, *J* = 7.4 Hz), 3.27 (t, 2H, *J* = 6.9 Hz, CH₂Br), 1.98-1.86 (m, 2H, CH₂), 1.84-1.74 (m, 2H, CH₂), 1.71 (quintet, 2H, *J* = 7.2 Hz, CH₂), 1.38-1.24 (m, 2H), 1.18-1.04 (m, 1H), 0.96-0.84 (m, 1H), 0.63 (t, 3H, *J* = 7.4 Hz, CH₃) ppm.

¹³C-NMR (CDCl₃, TMS, 101 MHz): 182.9, 141.4, 132.5, 127.6, 122.9, 122.4, 109.7, 54.2, 37.4, 33.6, 32.4, 31.0, 28.2, 23.4, 8.5 ppm.

| Elementary analysis for the Formula C₁₅H₂₀BrNO (310.24): | | | | |
|---|---|---|---|---|
| Calculated: | C 58.07, | H 6.50, | N 4.51, | Cl 25.76 %. |
| Found: | C 57.95, | H 6.42, | N 4.67, | Cl 25.58 %. |

### Example 10

### 3-(4-Chlorobutyl)-3-isobutyl-1,3-dihydro-2H-indol-2-one

The title compound is prepared according to process "A" starting from 3-isobutyl-1,3-dihydro-2*H*-indol-2-one and 1-bromo-4-chlorobutane.
M.p.: 124-125 °C (hexane-ethyl acetate).

IR (KBr): 3208, 1713, 747 cm⁻¹.

¹H-NMR (CDCl₃, TMS, 400 MHz): 9.02 (br s, 1H, NH), 7.21 (dt, 1H, *J* = 7.5, 1.4 Hz, H-6), 7.11 (td, 1H, *J* = 7.4, 0.6 Hz, H-4), 7.04 (dt, 1H, *J* = 7.4, 1.0 Hz, H-5), 6.95 (d, 1H, *J* = 7.7 Hz, H-7), 3.37 (t, 2H, *J* = 6.7 Hz, CH₂Cl), 1.95-1.70 (m, 4H, 2 x CH₂), 1.70-1.58 (m, 2H, CH₂), 1.38-1.30 (m, 1H), 1.23-1.17 (m, 1H), 1.02-0.98 (m, 1H), 0.73 (d, 3H, *J* = 6.6 Hz, CH₃), 0.61 (d, 3H, *J* = 6.6 Hz, CH₃) ppm.

¹³C-NMR (CDCl₃, TMS, 101 MHz): 183.1, 141.1, 132.6, 127.7, 123.3, 122.3, 109.8, 53.0, 46.3, 44.4, 39.2, 32.6, 25.3, 24.2, 23.6, 21.1 ppm.

| Elementary analysis for the Formula C₁₆H₂₂ClNO (279.81): | | | | |
|---|---|---|---|---|
| Calculated: | C 68.68, | H 7.93, | N 5.01, | Cl 12.67 %. |
| Found: | C 68.49, | H 7.89, | N 4.92, | Cl 12.89 %. |

### Example 11

### 3-(5-Bromopentyl)-3-ethyl-5-fluoro-1,3-dihydro-2H-indol-2-one

The title compound is prepared according to process "A" starting from 3-ethyl-5-fluoro-1,3-dihydro-2*H*-indol-2-one and 1,5-dibromopentane.
M.p.: 82-83 °C (hexane).

IR (KBr): 3293, 1720, 1690, 1175, 817 cm⁻¹.

¹H-NMR (CDCl₃, TMS, 400 MHz): 7.96 (br s, 1H, NH), 6.92 (dt, 1H, *J* = 8.8, 2.6 Hz, H-6), 6.86 (dd, 1H, *J* = 8.0, 2.6 Hz, H-4), 6.82 (dd, 1H, *J* = 8.4, 4.3 Hz, H-7), 3.30 (t, 2H, *J* = 6.9 Hz, CH₂Br), 1.96-1.87 (m, 2H, CH₂), 1.80-1.68 (m, 4H, 2 x CH₂), 1.40-1.25 (m, 2H, CH₂), 1.18-1.04 (m, 1H), 0.96-0.84 (m, 1H), 0.64 (t, 3H, *J* = 7.4 Hz, CH₃) ppm.

¹³C-NMR (CDCl₃, TMS, 101 MHz): 181.8, 159.3 (d, *J* = 240.7 Hz), 136.9, 134.4 (d, *J* = 8.0 Hz), 114.0 (d, *J* = 23.3 Hz), 111.0 (d, *J* = 24.4 Hz), 109.9 (d, *J* = 8.0 Hz), 54.7, 37.5, 33.6, 32.4, 31.1, 28.2, 23.5, 8.5 ppm.

| Elementary analysis form the Formula C₁₅H₁₉BrFNO (328.23): | | | | |
|---|---|---|---|---|
| Calculated: | C 54.89, | H 5.83, | N 4.27, | Br 24.34 %. |
| Found: | C 54.68, | H 5.89, | N 4.35, | Br 24.16 %. |

### Example 12

### 3-(5-Bromopentyl)-3-ethyl-5-methyl-1,3-dihydro-2H-indol-2-one

The title compound is prepared according to process "A" starting from 3-ethyl-5-methyl-1,3-dihydro-2*H*-indol-2-one and 1,5-dibromopentane.
M.p.: 72-73 °C (hexane).

IR (KBr): 3262, 1726,1694, 812 cm⁻¹.

¹H-NMR (CDCl₃, TMS, 400 MHz): 7.55 (br s, 1H, NH), 7.00 (d, 1H, *J* = 7.9 Hz, H-6), 6.92 (s, 1H, H-4), 6.75 (d, 1H, *J* = 7.8 Hz, H-7), 3.30 (t, 2H, *J* = 6.8 Hz, CH₂Br), 1.94-1.84 (m, 2H, CH₂), 1.79-1.68 (m, 4H, 2 x CH₂), 1.35-1.24 (m, 2H, CH₂), 1.24-1.13 (m, 1H), 0.93-0.84 (m, 1H), 0.63 (t, 3H, *J* = 7.4 Hz, CH₃) ppm.

¹³C-NMR (CDCl₃, TMS, 101 MHz): 181.8, 159.3 (d, *J* = 240.7 Hz), 136.9, 134.4 (d, *J* = 8.0 Hz), 114.0 (d, *J* = 23.3 Hz), 111.0 (d, *J* = 24.4 Hz), 109.9 (d, *J* = 8.0 Hz), 54.7, 37.5, 33.6, 32.4, 31.1, 28.2, 23.5, 8.5 ppm.

| Elementary analysis for the Formula C₁₆H₂₂BrNO (324.26): | | | | |
|---|---|---|---|---|
| Calculated: | C 59.27, | H 6.84, | N 4.32, | Br 24.64 %. |
| Found: | C 59.18, | H 6.92, | N 4.55, | Br 24.51 %. |

### Example 13

### 3-(5-Bromopentyl)-3-ethyl-6-fluoro-1,3-dihydro-2H-indol-2-one

The title compound is prepared according to process "A" starting from 3-ethyl-6-fluoro-1,3-dihydro-2*H*-indol-2-one and 1,5-dibromopentane.
M.p.: 95-96 °C (hexane).

IR (KBr): 3300, 1722, 857 cm⁻¹.

¹H-NMR (CDCl₃, TMS, 400 MHz): 9.24 (br s, 1H, NH), 7.01 (dd, 1H, *J* = 8.1, 5.3 Hz, H-5), 6.72 (ddd, 1H, *J* = 9.6, 8.2, 2.3 Hz, H-5), 6.68 (d, 1H, *J* = 8.8, 2.3 Hz, H-7), 3.26 (t, 2H, *J* = 7.4 Hz, CH₂Br), 1.92-1.83 (m, 2H, CH₂), 1.80-1.65 (m, 4H, 2 x CH₂), 1.35-1.25 (m, 2H, CH₂), 1.09-1.00 (m, 1H), 0.92-0.84 (m, 1H), 0.60 (t, 3H, *J* = 7.4 Hz, CH₃) ppm.

¹³C-NMR (CDCl₃, TMS, 101 MHz): 183.3, 162.4 (d, *J* = 244.1 Hz), 142.5 (d, *J* = 11.8 Hz), 127.7 (d, *J* = 3.1 Hz), 123.8 (d, *J* = 9.9 Hz), 108.7 (d, *J* = 22.1 Hz), 98.4 (d, *J* = 27.1 Hz), 53.9, 37.4, 33.6, 32.3, 31.0, 28.2, 23.4, 8.4 ppm.

| Elementary analysis for the Formula C₁₅H₁₉BrFNO (328.23): | | | | | |
|---|---|---|---|---|---|
| Calculated: | C 54.89, | H 5.83, | N 4.27, | Br 24.34 % | F 5.79 %. |
| Found: | C 54.69, | H 5.67, | N 4.39, | Br 24.19%. | |

### Chlorination of ω-haloalkyl compounds in position 5 (Process "B")

The haloalkyl compound (5 mmoles) is dissolved in 15 ml of glacial acetic acid, the solution is cooled until glacial acetic acid begins to separate (14-16 °C) and a solution of 0.5 ml (5.7 mmoles) of sulfuryl chloride in 5 ml of glacial acetic acid is dropped to it. The mixture is stirred for 2 hours at the same temperature and then pipetted onto ice-water. The separated white substance is filtered, washed with water and hexane, dried and used for the coupling reaction without purification. Analytical samples may be obtained by recrystallization from the indicated solvent.

### Example 14

### 5-Chloro-3-(4-chlorobutyl)-3-ethyl-1,3-dihydro-2H-indol-2-one

The title compound is prepared according to process "B" starting from 3-(4-chlorobutyl)-3-ethyl-1,3-dihydro-2*H*-indol-2-one.
M.p.: 116-117 °C (hexane-ethyl acetate).

IR (KBr): 3285, 1717, 818 cm⁻¹.

¹H-NMR (CDCl₃, TMS, 400 MHz): 8.72 (br s, 1H, NH), 7.15 (dd, 1H, *J* = 8.2, 2.1 Hz, H-6), 7.12 (d, 1H, *J* = 2.1 Hz, H-4), 6.86 (d, 1H, *J* = 8.2 Hz, H-7), 3.41 (t, 2H, *J* = 6.7 Hz, CH₂Cl), 2.00-1.86 (m, 2H, CH₂), 1.84-1.74 (m, 2H, CH₂), 1.74-1.60 (m, 2H), 1.29-1.15 (m, 1H), 1.12-0.95 (m, 1H), 0.65 (t, 3H, *J* = 7.4 Hz, CH₃) ppm.

¹³C-NMR (CDCl₃, TMS, 101 MHz): 182.0, 139.8, 134.2, 127.9, 127.8, 123.4, 110.7, 54.5, 44.4, 36.8, 32.5, 31.0, 21.7, 8.5 ppm.

| Elementary analysis for the Formula C₁₄H₁₇Cl₂NO (286.20): | | | | |
|---|---|---|---|---|
| Calculated: | C 58.75, | H 5.99, | N 4.89, | Cl 24.77 %. |
| Found: | C 58.61, | H 5.96, | N 4.80, | Cl 24.66 %. |

### Example 15

### 5-Chloro-3-(3-chloropropyl)-3-ethyl-1,3-dihydro-2H-indol-2-one

The title compound is prepared according to process "B" starting from 3-(3-chloropropyl)-3-ethyl-1,3-dihydro-2*H*-indol-2-one.
M.p.: 105-107 °C (hexane).

IR (KBr): 3221, 2963, 1700 (C=O), 1677, 1474 cm⁻¹.

¹H-NMR (CDCl₃, TMS, 400 MHz): 9.15 (br s, 1H, NH), 7.21 (dd, 1H, *J* = 8.2, 2.1 Hz, H-6), 7.12 (d, 1H, *J* = 2.0 Hz, H-4), 6.88 (d, 1H, *J* = 8.2 Hz, H-7), 3.43-3.39 (m, 2H, CH₂Cl), 2.10-1.77 (m, 4H, 2 x CH₂), 1.62-1.55 (m, 1H), 1.42-1.38 (m, 1H), 0.66 (t, 3H, *J* = 7.4 Hz, CH₃) ppm.

¹³C-NMR (CDCl₃, TMS, 101 MHz): 182.1, 139.8, 133.9, 128.1, 128.0, 123.5, 110.8, 54.1, 44.6, 34.7, 30.9, 27.5, 8.5 ppm.

| Elementary analysis for the Formula C₁₃H₁₅Cl₂NO (272.18): | | | | |
|---|---|---|---|---|
| Calculated: | C57.37, | H 5.56, | N 5.15, | Cl 26.05 %. |
| Found: | C 57.19, | H 5.64, | N 5.28, | Cl 25.88 %. |

### Example 16

### 5-Chloro-3-(4-chlorobutyl)-3-ethyl-6-fluoro-1,3-dihydro-2H-indol-2-one

The title compound is prepared according to process "B" starting from 6-fluoro-3-(4-chlorobutyl)-3-ethyl-1,3-dihydro-2*H*-indol-2-one.
M.p.: 131-133 °C (hexane-ethyl acetate).

IR (KBr): 3289, 1720, 1143 cm⁻¹.

¹H-NMR (CDCl₃, TMS, 400 MHz): 8.90 (br s, 1H, NH), 7.12 (d, 1H, *J* = 7.1, H-4), 6.79 (d, 1H, *J* = 8.8 Hz, H-7), 3.42 (t, 2H, *J* = 6.7 Hz, CH₂Cl), 1.96-1.84 (m, 2H, CH₂), 1.80-1.63 (m, 4H, 2 x CH₂), 1.30-1.20 (m, 1H), 1.20-1.04 (m, 1H), 0.65 (t, 3H, *J* = 7.4 Hz, CH₃) ppm.

¹³C-NMR (CDCl₃, TMS, 101 MHz): 182.3, 157.6 (d, *J* = 247.2 Hz), 140.9 (d, *J* = 11.1 Hz), 128.8 (d, *J* = 3.8 Hz), 124.8, 114.3 (d, *J* = 18.3 Hz), 99.5 (d, *J* = 26.7 Hz), 54.2, 44.3, 36.8, 32.4, 31.0, 21.6, 8.4 ppm.

| Elementary analysis for the Formula C₁₄H₁₆Cl₂FNO (304.19): | | | | |
|---|---|---|---|---|
| Calculated: | C 55.28, | H 5.30, | N4.60, | Cl 23.31 %. |
| Found: | C 55.19, | H 5.27, | N 4.58, | Cl 23.34 %. |

### 5,7-Dichlorination of ω-chloroalkyl compounds (Process "C")

The chloroalkyl compound is dissolved in 80 ml (40 mmoles) of glacial acetic acid, 9.6 ml (120 mmoles) of sulfuryl chloride are dropped to it at room temperature and the solution is kept at 60 °C for 3 hours. Then the reaction mixture is cooled, poured onto ice and extracted with diethyl ether. The ether phase is extracted twice with 10 % by volume NaOH solution, dried over sodium sulfate and evaporated. The thus-obtained pale yellow oil is triturated with hexane, the white substance separated in crystalline form is stirred in hexane, filtered, washed with hexane, dried again and used for the coupling reaction without purification. Analytical samples may be obtained from the given compounds by recrystallization from the indicated solvents.

### Example 17

### 5,7-Dichloro-3-(4-chlorobutyl)-3-ethyl-1,3-dihydro-2H-indol-2-one

The title compound is prepared according to process "C" starting from 3-(4-chlorobutyl)-3-ethyl-1,3-dihydro-2*H*-indol-2-one.
M.p.: 65-67 °C (hexane).

IR (KBr): 3165, 2964, 1713 (C=O), 1455 cm⁻¹.

¹H-NMR (CDCl₃, TMS, 400 MHz): 8.38 (br s, 1H, NH), 7.20 (d, 1H, *J* = 1.9 Hz, H-6), 6.97 (d, 1H, *J* = 1.8 Hz, H-4), 3.38 (t, 2H, *J* = 6.7 Hz, CH₂Cl), 1.95-1.84 (m, 2H, CH₂), 1.76-1.60 (m, 4H, 2 x CH₂), 1.19-1.16 (m, 1H), 1.04-0.96 (m, 1H), 0.62 (t, 3H, *J* = 7.4 Hz, CH₃) ppm.

¹³C-NMR (CDCl₃, TMS, 101 MHz): 180.5, 137.7, 135.1, 128.3, 127.6, 121.9, 115.7, 55.7, 44.3, 36.8, 32.5, 31.0, 21.7, 8.5 ppm.

| Elementary analysis for the Formula C₁₆H₂₂ClNO (279.81): | | | | |
|---|---|---|---|---|
| Calculated: | C 52.44, | H 5.03, | N 4.37, | Cl 33.17 %. |
| Found: | C 52.37, | H 4.97, | N 4.27, | Cl 33.18 %. |

### Example 18

### 5,7-Dichloro-3-(4-chlorobutyl)-3-isobutyl-1,3-dihydro-2H-indol-2-one

The title compound is prepared according to process "C" starting from 3-(4-chlorobutyl)-3-isobutyl-1,3-dihydro-2*H*-indol-2-one.
M.p.: 93-94 °C (hexane).

IR (KBr): 3144, 1719, 1459 cm⁻¹.

¹H-NMR (CDCl₃, TMS, 400 MHz): 8.49 (br s, 1H, NH), 7.24 (dt, 1H, *J* = 1.9 Hz, H-6), 7.01 (d, 1H, *J* = 1.7 Hz, H-4), 3.41 (t, 2H, *J* = 6.7 Hz, CH₂Cl), 1.91 (m, 2H, CH₂), 1.67 (m, 4H, 2 x CH₂), 1.34 (m, 1H), 1.20 (m, 1H), 1.01 (m, 1H), 0.74 (d, 3H, *J* = 6.7 Hz, CH₃), 0.66 (d, 3H, *J* = 6.7 Hz, CH₃) ppm.
¹³C-NMR (CDCl₃, TMS, 101 MHz): 181.0, 137.5, 135.4, 128.2, 127.6, 122.2, 115.4, 54.5, 46.3, 44.3, 39.2, 32.4, 25.3, 24.3, 23.1, 21.1 ppm.

| Elementary analysis for the Formula C₁₆H₂₂ClNO (279.81): | | | | |
|---|---|---|---|---|
| Calculated: | C 55.11, | H 5.78, | N 4.02, | Cl 30.50 %. |
| Found: | C 55.29, | H 5.67, | N 4.12, | Cl 30.18 %. |

### Example 19

### 7-Chloro-3-(4-chlorobutyl)-3-ethyl-5-fluoro-1,3-dihydro-2H-indol-2-one

3-(4-Chlorobutyl)-3-ethyl-5-fluoro-1,3-dihydro-2*H*-indol-2-one (5.40 g; 20 mmoles) is dissolved in 40 ml of glacial acetic acid, 3.2 ml (40 mmole) of sulfuryl chloride are dropped to it at room temperature, and the solution is kept at 60 °C for 4 hours. The reaction mixture is then cooled, poured onto ice and extracted with diethyl ether. The ether phase is extracted twice with 10 % by volume NaOH solution, dried over sodium sulfate and evaporated. The thus-obtained pale yellow oil is triturated with hexane, the white substance separated in crystalline form is stirred in hexane, filtered, washed with hexane, dried and used for the coupling reaction without purification. Analytical samples may be obtained by recrystallization from the mixture of hexane and ethyl acetate.
M.p.: 104 - 105 °C (hexane-ethyl acetate).

IR (KBr): 3184, 1709, 1080, 853 cm⁻¹.

¹H-NMR (CDCl₃, TMS, 400 MHz): 8.22 (br s, 1H, NH), 6.99 (dd, 1H, *J* = 7.6, 2.3 Hz), 6.81 (dd, 1H, *J* = 7.6, 2.3 Hz), 3.42 (t, 2H, *J* = 6.7 Hz, CH₂Cl), 2.00-1.88 (m, 2H, CH₂), 1.82-1.60 (m, 4H, 2 x CH₂), 1.30-1.16 (m, 1H), 1.12-1.00 (m, 1H), 0.66 (t, 3H, *J* = 7.4 Hz, CH₃)

¹³C-NMR (CDCl₃, TMS, 101 MHz): 180.6, 158.8 (d, *J* = 244.5 Hz), 135.1 (d, *J* = 2.3 Hz), 134.9 (d, *J* = 8.4 Hz), 114.8 (d, *J* = 26.3 Hz), 114.8 (d, *J* = 11.0 Hz), 109.7 (d, *J =* 24.4 Hz), 55.8 (d, *J* = 1.9 Hz), 44.3, 36.8, 32.5, 31.1, 21.7, 8.5.

| Elementary analysis for the Formula C₁₆H₂₂ClNO (279.81): | | | | |
|---|---|---|---|---|
| Calculated: | C 55.28, | H 5.30, | N 4.60, | Cl 23.31 %. |
| Found: | C 55.19, | H 5.28, | N 4.65, | Cl 23.19 %. |

### Example 20

### 5-Bromo-3-(4-chlorobutyl)-3-ethyl-1,3-dihydro-2H-indol-2-one

3-(4-Chlorobutyl)-3-ethyl-1,3-dihydro-2*H*-indol-2-one (12.59 g; 50 mmoles) is dissolved in the mixture of 100 ml of dioxane and 100 ml of water. A mixture of 2.84 ml of bromine (55 mmoles), 11.9 g of KBr (100 mmoles) and 50 ml of water is dropped to the solution at a temperature between 80 °C and 90 °C within half an hour. The reaction mixture is kept at the same temperature for further half an hour and allowed to cool. Then 500 ml of water is dropped to it. The product separates in form of white crystals. The separated substance is filtered off, washed with water and hexane and used for the coupling reaction without purification. Analytical samples may be obtained by recrystallization from a mixture of hexane and ethyl acetate.
M.p.: 117-118 °C (hexane-ethyl acetate).

IR (KBr): 3286, 1717, 1198, 817 cm⁻¹.

¹H-NMR (CDCl₃, TMS, 400 MHz): δ 9.28 (br s, 1H, NH), 7.35 (dd, 1H, *J* = 8.2, 2.0 Hz, H-6), 7.24 (d, 1H, *J* = 2.0 Hz, H-4), 6.84 (d, 1H, *J* = 8.2 Hz, H-7), 3.41 (t, 2H, *J* = 6.8 Hz, CH₂Cl), 1.98-1.75 (m, 2H, CH₂), 1.74-1.60 (m, 4H, 2 x CH₂), 1.27-1.16 (m, 1H), 1.11-1.01 (m, 1H), 0.64 (t, 3H, *J* = 7.4 Hz, CH₃);

¹³C-NMR (CDCl₃, TMS, 101 MHz): 182.3, 140.4, 134.6, 130.7, 126.1, 115.3, 111.3, 54.5, 44.3, 36.7, 32.8, 30.9, 21.7, 8.5.

| Elementary analysis for the Formula C₁₄H₁₇BrClNO (330.65): | | | |
|---|---|---|---|
| Calculated: | C 50.86, | H 5.18, | N 4.24 %., |
| Found: | C 50.79, | H 5.09, | N 4.38 %., |

### Example 21

### 3-(4-Chlorobutyl)-3-ethyl-2-oxoindolin-5-sulfonyl chloride

90 ml of chlorosulfonic acid are cooled to 0 °C, and 3-(4-chlorobutyl)-3-ethyl oxindole (11.34 g; 45 mmoles) is added to it in portion so that the temperature does not exceed 2 °C. The solution is then allowed to worm up to room temperature under stirring, pipetted onto ice in half an hour, the separated white precipitate is filtered off, washed with water and hexane and used for the coupling reaction without purification. Analytical samples may be obtained by recrystallization from a mixture of hexane and ethyl acetate.
M.p.: 141 - 143 °C.

IR (KBr): 3197, 1729, 1371,1176 cm⁻¹.

¹H-NMR (CDCl₃, TMS, 400 MHz): 9.39 (br s, 1H, NH), 7.99 (dd, 1H, *J* = 8.4, 1.9 Hz, H-6), 7.80 (d, 1H, *J* = 1.9 Hz, H-4), 7.16 (d, 1H, *J* = 8.4 Hz, H-7), 3.46-3.41 (m, 2H, CH₂Cl), 2.10-1.83 (m, 4H, 2 x CH₂), 1.73-1.66 (m, 2H), 1.32-1.18 (m, 1H), 1.14-1.00 (m, 1H), 0.68 (t, 3H, *J* = 7.4 Hz, CH₃) ppm.

¹³C-NMR (CDCl₃, TMS, 101 MHz): 182.4, 147.6, 138.4, 133.9, 128.8, 121.9, 110.1, 54.5, 44.2, 36.4, 32.2, 30.9, 21.5, 8.5 ppm.

| Analysis for the Formula C₁₄H₁₇Cl₂NO₃S (350.27): | | | | | |
|---|---|---|---|---|---|
| Calculated: | C 48.01, | H 4.89, | N 4.00, | Cl 20.24 | S 9.15 %. |
| Found: | C 47.89, | H 4.76, | N 4.18, | Cl 20.01 | S 9.38 %. |

### Example 22

### 3-(4-Chlorobutyl)-3-ethyl-2-oxoindoline-5-sulfonamide

3-(4-Chlorobutyl)-3-ethyl-2-oxoindoline-5-sulfonil chloride (9.96 g; 30 mmoles) is dissolved in 450 ml of ethanol, and 25 % aqueous ammonia solution (9 ml, 120 mmoles) is dropped to the solution at 0-2 °C. The mixture is then allowed to warm up to room temperature and stirred further for 1 hour. The solution is then evaporated, the residual white substance is stirred in water, filtered, washed with water and hexane and used for the coupling reaction without purification. Analytical samples may be obtained by recrystallization from ethyl acetate.
M.p.: 171-172 °C (ethyl acetate).

IR (KBr): 3343, 3265, 1725, 1327, 1169 cm⁻¹.

¹H-NMR (DMSO-*d*₆, TMS, 400 MHz): 10.8 (br s, 1H, NH), 7.70 (dd, 1H, *J* = 8.1, 1.8 Hz, H-6), 7.65 (d, 1H, *J* = 1.7 Hz, H-4), 6.98 (d, 1H, *J* = 8.1 Hz, H-7), 3.54-3.49 (m, 2H, CH₂Cl), 1.82-1.73 (m, 4H, 2 x CH₂), 1.59 (quintet, 2H, *J* = 7.2 Hz, CH₂), 1.15-1.00 (m, 1H), 1.00-0.85 (m, 1H), 0.52 (t, 3H, *J* = 7.4 Hz, CH₃) ppm.
¹³C-NMR (DMSO-*d*₆, TMS, 101 MHz): 181.0, 145.7, 137.6, 132.6, 126.6, 120.9, 109.1, 53.4, 45.1, 36.2, 32.3, 30.3, 21.5, 8.5 ppm.

| Elementary analysis for the Formula C₁₄H₁₉ClN₂O₃S (330.84): | | | | | |
|---|---|---|---|---|---|
| Calculated: | C 50.83, | H 5.79, | N 8.47, | Cl 10.72 | S 9.69 %. |
| Found: | C 50.79, | H 5.74, | N 8.51, | Cl 10.71 | S 9.72 %. |

### Coupling reactions of ω-chloroalkyl compounds (Process "D")

In the coupling reaction the appropriate chloroalkyl compound is coupled with the secondary amine. The melt of the base (12 mmoles) is warmed to 180 °C under slow stirring, and the chloroalkyl compound (12 mmoles) and the sodium carbonate (1.36 g; 12 mmoles) are added to it at the same temperature. The mixture is reacted for 1 hour, allowed to cool, ethyl acetate and water are added to it and the phases are separated. The organic phase is evaporated, the residual oil is subjected to chromatography using a short column and ethyl acetate as eluent. The desired compounds are prepared as main products.

### Process "D", processing method 1

If the product purified by column chromatography gets crystalline upon trituration with diethyl ether, it is filtered off and recrystallized from the solvent indicated after the melting point of the given substance. The desired compounds are obtained in form of white crystals.
Process "D", processing method 2 If the basic product does not get crystalline upon the addition of diethyl ether, it is dissolved in 200 ml of ether, the slight amount of floating precipitate is filtered off, and to the pure solution a solution of the calculated amount (one molar equivalent) of hydrogen chloride in 50 ml of diethyl ether is added under vigorous stirring. The separated white salt is filtered, washed with ether and hexane and dried in a vacuum pistol at room temperature for 3 hours. If necessary, the hydrochloride salt is recrystallized.
Process "D", processing method 3 If the basic product does not get crystalline upon the addition of diethyl ether and does not provide a well-filterable salt with hydrogen chloride, it is dissolved in 100 ml of hot ethyl acetate, and a solution of 1 molar equivalent of oxalic acid dihydrate in 50 ml of hot ethyl acetate is dropped to it within 10 minutes, under stirring. The white oxalate salt separates upon cooling. It is filtered off at room temperature, washed with ethyl acetate and hexane and dried.

### Example 23

### 3-[3-(6,7-Dihydro-4H-thieno[3,2-c]pyridin-5-yl)-propyl]-3-ethyl-1,3-dihydro-2H-indol-2-one

The title compound is prepared according to process "D" by applying processing method 1 starting from 3-(3-chloropropyl)-3-ethyl-1,3-dihydro-2*H*-indol-2-one and 2-chloro-6,7-dihydro-4*H-*thieno[3,2-c]pyridine.
M.p.: 130-132 °C (hexane-ethyl acetate).

IR (KBr): 3107, 3059, 1706 (C=O) cm⁻¹.

¹H-NMR (CDCl₃, TMS, 400 MHz): 0.63 (3H, t, *J* = 7.4 Hz), 1.22-1.10 (1H, m), 1.46-1.30 (1H, m), 1.90-1.77 (2H, m), 1.98-1.90 (2H, m), 2.42 (2H, t, *J* = 7.4 Hz), 2.64 (2H, t, *J* = 5.7 Hz), 2.81 (2H, t, *J* = 5.4 Hz), 3.32 (1H, d, *J* = 14.4 Hz), 3.42 (1H, d, *J* = 14.4 Hz), 6.65 (1H, d, *J* = 5.2 Hz), 6.87 (1H, d, *J* = 7.7 Hz), 7.03 (1H, d, *J* = 5.2 Hz), 7.04 (1H, dt, *J* = 1.0, 7.5 Hz), 7.12 (1H, dd, *J* = 0.6, 7.3 Hz), 7.18 (1H, dt, *J* = 1.3, 7.6 Hz), 8.80 (1H, s) ppm.

¹³C-NMR (CDCl₃, TMS, 101 MHz): 182.5, 141.4, 133.7, 133.3, 132.5, 127.6, 125.2, 123.0, 122.6, 122.3, 109.5, 57.5, 54.0, 52.9, 50.7, 35.3, 31.0, 25.4, 22.1, 8.6 ppm.

| Analysis for the Formula C₂₀H₂₄N₂OS (340.49): | | | | |
|---|---|---|---|---|
| Calculated: | C 70.55, | H 7.10, | N 8.23, | S 9.42 %. |
| Found: | C 69.20, | H 7.10, | N 8.03, | S 9.10 %. |

### Example 24

### 5-Chloro-3-[3-(6,7-dihydro-4H-thieno[3,2-c]-pyridin-5-yl)-propyl]-3-ethyl-1,3-dihydro-2H-indol-2-one

The title compound is prepared according to process "D" by applying processing method 1 starting from 5-chloro-3-(3-chloropropyl)-3-ethyl-1,3-dihydro-2*H*-indol-2-one and 2-chloro-6,7-dihydro-4*H*-thieno[3,2-c]-pyridine.
M.p.: 66-69 °C (hexane-ethyl acetate).

IR (KBr): 1652 (C=O) cm⁻¹.

¹H-NMR (CDCl₃, TMS, 400 MHz): 0.62 (3H, t, *J* = 7.4 Hz), 1.18-1.14 (1H, m), 1.40-1.36 (1H, m), 1.85-1.74 (2H, m), 1.98-1.90 (2H, m), 2.47-2.40 (2H, m), 2.67 (2H, t, *J* = 5.5 Hz), 2.83 (2H, t, *J* = 5.5 Hz), 3.42 (2H, s), 6.67 (1H, d, *J* = 5.2 Hz), 6.74 (1H, d, *J* = 8.2 Hz), 7.03 (1H, d, *J* = 5.1 Hz), 7.09 (1H, d, *J* = 2.1 Hz), 7.15 (1H, dd, *J* = 2.2, 8.1 Hz), 9.39 (1H, s) ppm.

¹³C-NMR (CDCl₃, TMS, 101 MHz): 182.2, 140.1, 134.4, 133.6, 133.3, 127.7, 127.7,125.6, 123.3, 122.6, 110.5, 57.4, 54.5, 52.9, 50.8, 35.1, 31.0, 25.3, 22.1, 8.5 ppm.

| Elementary analysis for the Formula C₂₀H₂₃ClN₂OS (374.94): | | | | | |
|---|---|---|---|---|---|
| Calculated: | C 64.07, | H 6.18, | Cl 9.46, | N 7.47, | S 8.55 %. |
| Found: | C 63.96, | H 6.20, | Cl 9.17, | N 7.26 | S 8.45 %. |

### Example 25

### 3-Ethyl-3-{4-[4-(3-trifluoromethyl-phenyl)-1,2,3,6-tetrahydropyridin-1-yl]-butyl}-1,3-dihydro-2H-indol-2-one monooxalate

The title compound is prepared according to process "D" by applying processing method 3 starting from 3-(4-chlorobutyl)-3-ethyl-1,3-dihydro-2*H*-indol-2-one and 4-(3-trifluoromethylphenyl)-1,2,3,6-tetrahydropyridine.
M.p.: 136-139 °C.

IR (KBr): 3185, 1707 (C=O) cm⁻¹.

¹H-NMR (DMSO-*d₆*, TMS, 400 MHz): 10.4 (1H, s), 7.77 (1H, d), 7.75 (1H, s), 7.67 (1H, d, *J* = 7.7 Hz), 7.61 (1H, t, *J* = 7.7 Hz), 6.30 (1H, s), 5.2 (4H, br s), 3.69 (2H, s), 3.22 (2H, s), 2.90 (2H, t, *J* = 8.0 Hz), 2.73 (2H, s), 1.80-1.66 (4H, m), 1.62-1.48(2H, m), 1.06-0.94 (1H, m), 0.88-0.76 (1H, m), 0.51 (3H, t, *J* = 7.4 Hz) ppm.

¹³C-NMR (DMSO-*d*₆, TMS, 101 MHz): 180.9, 164.6, 142.7, 139.9, 139.1, 132.2, 129.9, 129.6 (q, *J* = 31.7 Hz), 129.1, 127.8, 124.5 (q, *J* = 3.8 Hz), 124.4 (q, *J* = 272.4 Hz), 123.2, 121.7, 121.5 (q, *J* = 3.8 Hz), 110.4, 109.4, 54.8, 53.2, 49.9, 48.2, 36.7, 30.4, 24.1, 24.0, 21.6, 8.6 ppm.

| Elementary analysis for the Formula C₂₈H₃₁F₃N₂O₅ (532.56): | | | |
|---|---|---|---|
| Calculated: | C 63.15, | H 5.87, | N 5.26 %., |
| Found: | C 62.72, | H 5.92, | N 5.22 %., |

### Example 26

### 5-Chloro-3-[4-(6,7-dihydro-4H-thieno[3,2-c]-pyridin-5-yl)-butyl]-3-ethyl-1,3-dihydro-2H-indol-2-one monohydrochloride

The title compound is prepared according to process D by applying processing method 1 starting from 5-chloro-3-(4-chlorobutyl)-3-ethyl-1,3-dihydro-2*H*-indol-2-one and 6,7-dihydro-4*H*-thieno[3,2-c]pyridine.
M.p.: 213-215 °C.

IR (KBr): 3186, 2473, 1708 (C=O) cm⁻¹.

¹H-NMR (DMSO-*d*₆, TMS, 400 MHz): 0.51 (3H, t, *J* = 7.4 Hz), 0.90-0.75 (1H, m), 1.02-0.90 (1H, m), 1.87-1.69 (6H, m), 3.05 (4H, br s), 3.26 (1H, br s), 3.36 (1H, br s), 4.09 (1H, br s), 4.34 (1H, br s), 6.88 (1H, d, *J* = 8.1 Hz), 6.88 (1H, d, *J* = 5.2 Hz), 7.23 (1H, dd, *J* = 2.1, 8.2 Hz), 7.36 (1H, d, *J* = 2.0 Hz), 7.45 (1H, d, *J* = 5.2 Hz), 10.6 (1H, s), 11.1 (1H, br s) ppm.

¹³C-NMR (DMSO-*d*₆, TMS, 101 MHz): 180.4, 141.6, 134.4, 131.6, 128.3, 127.7, 125.9, 125.3, 125.2, 123.5, 110.7, 54.6, 53.8, 50.0, 49.1, 36.4, 30.2, 23.6, 21.7, 21.4, 8.5 ppm.
Elementary analysis for the Formula C₂₁H₂₆Cl₂NO₂S (425.42):
Calculated: C 59.29, H 6.16, Cl 16.67, N 6.58 %.
Found: C 58.83, H 6.17, Cl 16.26, N 6.43 %.

### Example 27

### 5-Bromo-3-[4-(6,7-dihydro-4H-thieno[3,2-c]pyridin-5-yl)-butyl]-3-ethyl-1,3-dihydro-2H-indol-2-one

The title compound is prepared according to process D by applying processing method 1 starting from 5-bromo-3-(4-chlorobutyl)-3-ethyl-1,3-dihydro-2*H*-indol-2-one and 6,7-dihydro-4*H*-thieno[3,2-c]pyridine.
M.p.: 149-151 °C (hexane-ethyl acetate).

IR (KBr): 3444, 3110, 1720 (C=O) cm⁻¹.

¹H-NMR (CDCl₃, TMS, 400 MHz): 0.62 (3H, t, *J* = 7.4 Hz), 0.98-0.86 (1H, m), 1.18-1.04 (1H, m), 1.52-1.45 (2H, m), 1.80-0.71 (2H, m), 1.97-1.88 (2H, m), 2.41 (2H, t, *J* = 7.6 Hz), 2.71 (2H, t, *J* = 5.6 Hz), 2.83 (2H, t, *J* = 5.6 Hz), 3.47 (2H, s), 6.72 (1H, d, *J* = 8.2 Hz), 6.68 (1H, d, *J* = 5.1 Hz), 7.04 (1H, d, *J* = 5.1 Hz), 7.22 (1H, d, *J* = 2.0 Hz), 7.30 (1H, dd, *J* = 2.0, 8.2 Hz), 9.34 (1H, s) ppm.

¹³C-NMR (CDCl₃, TMS, 101 MHz): 182.3, 140.6, 134.9, 133.7, 133.3, 130.5, 126.1, 125.2, 122.6, 115.0, 111.1, 57.3, 54.6, 52.9, 50.7, 37.5, 37.5, 31.0, 25.3, 22.2, 8.5 ppm.

| Elementary analysis for the Formula C₁₆H₂₂ClNO (279.81): | | | | | |
|---|---|---|---|---|---|
| Calculated: | C 58.20, | H 5.81, | Br 18.44, | N 6.46, | S 7.40 %. |
| Found: | C 58.59, | H 5.92, | Br 18.01 | N 6.31, | S 7.16 %. |

### Example 28

### 3-[4-(6,7-Dihydro-4H-thieno[3,2-c]pyridin-5-y1)-butyl]-3-isobutyl-1,3-dihydro-2H-indol-2-one

The title compound is prepared according to process "D" by applying processing method 1 starting from 3-(4-chlorobutyl)-3-isobutyl-1,3-dihydro-2*H*-indol-2-one and 6,7-dihydro-4*H-*thieno[3,2-c]pyridine.
M.p.: 114-116 °C (hexana-ethyl acetate).

IR (KBr): 3201, 1718 (C=O) cm⁻¹.

¹H-NMR (CDCl₃, TMS, 400 MHz): 0.60 (3H, d, *J* = 6.7 Hz), 0.72 (3H, d, *J* = 6.7 Hz), 0.90-0.86 (1H, m), 1.16-1.04 (1H, m), 1.38-1.25 (1H, m), 1.48-1.40 (2H, m), 1.80-1.68 (2H, m), 1.94-1.88 (2H, m), 2.37 (2H, t, *J* = 7.8 Hz), 2.68 (2H, t, *J* = 5.7 Hz), 2.81 (2H, t, *J* = 5.5 Hz), 3.45 (2H, s), 6.67 (1H, d, *J* = 5.1 Hz), 6.89(1H, d, *J* = 7.7 Hz), 7.02 (1H, dt, *J* = 0.9, 7.5 Hz), 7.03 (1H, d, *J* = 5.1 Hz), 7.10 (1H, d, *J* = 6.8 Hz), 7.18 (1H, dt, *J* = 1.2, 7.7 Hz), 9.06 (1H, s) ppm.

¹³C-NMR (CDCl₃, TMS, 101 MHz): 183.2, 141.2, 133.7, 133.3, 132.9, 127.5, 125.2, 123.3, 122.5, 122.2, 109.6, 57.4, 53.1, 53.0, 50.0, 46.3, 40.0, 27.4, 25.3, 24.2, 23.1, 21.7, 21.7 ppm.

| Elementary analysis for the Formula C₂₃H₃₀N₂OS (382.57): | | | | |
|---|---|---|---|---|
| Calculated: | C 72.21, | H 7.90, | N 7.32, | S 8.38 %. |
| Found: | C 71.17, | H 8.18, | N 7.07, | S 8.21 %. |

### Example 29

### 3-[4-(6,7-Dihydro-4H-thieno[3,2-c]pyridin-5-yl)-butyl]-3-ethyl-1,3-dihydro-2H-indol-2-one monohydrochloride

The title compound is prepared according to process "D" by applying processing method 2 starting from 3-(4-chlorobutyl)-3-isobutyl-1,3-dihydro-2*H*-indol-2-one and 6,7-dihydro-4*H-*thieno[3,2-c]pyridine.
M.p.: 143-144 °C.

IR (KBr): 3427, 1706 (C=O) cm⁻¹.

¹H-NMR (DMSO-*d₆*, TMS, 400 MHz): 11.2 (1H, br s), 10.5 (1H, s), 7.48 (1H, d, *J* = 5.1 Hz), 7.25 (1H, d, *J* = 7.2 Hz), 7.21 (1H, dt, *J* = 1.2, 7.6 Hz), 7.03 (1H, dt, *J* = 1.0, 7.5 Hz), 6.91 (1H, d, *J* = 5.3 Hz), 6.90 (1H, d, *J* = 7.8 Hz), 4.37 (1H, br s), 4.11 (1H, br s), 3.63 (1H, br s), 3.25 (1H, br s), 3.20 (1H, br s), 3.07 (3H, br s), 1.83-1.72 (6H, m), 1.01 (1H, br s), 0.85 (1H, br s), 0.54 (3H, t, *J* = 7.4 Hz) ppm.

¹³C-NMR (DMSO-*d₆*, TMS, 50 MHz): 180.7, 142.7, 132.1, 131.5, 128.2, 127.7, 125.3, 123.2, 121.6, 109.3, 54.6, 53.1, 49.9, 49.0, 36.5, 30.3, 23.6, 21.7, 21.4, 8.5 ppm.

| Elementary analysis for the Formula C₂₁H₂₇ClN₂OS (390.98): | | | | | |
|---|---|---|---|---|---|
| Calculated: | C 64.51, | H 6.96, | Cl 9.07, | N 7.16 | S 8.20 %. |
| Found: | C 64.44, | H 7.00, | Cl 8.87, | N 7.07 | S 8.04 %. |

### Example 30

### 3-[4-(6,7-Dihydro-4H-thieno[3,2-c]pyridin-5-yl)-butyl]-3-ethyl-5-methyl-1,3-dihydro-2H-indol-2-one

The title compound is prepared according to process "D" by applying processing method 1 starting from 3-(4-chlorobutyl)-3-ethyl-5-methyl-1,3-dihydro-2H-indol-2-one and 6,7-dihydro-4*H-*thieno[3,2-c]pyridine.
M.p.: 138-140 °C (hexane-ethyl acetate).

IR (KBr): 3239, 1710 (C=O), 1493 cm⁻¹.

¹H-NMR (CDCl₃, TMS, 400 MHz): 7.90 (1H, br s), 7.02 (1H, d, *J* = 5.1 Hz), 6.97 (1H, m), 6.90 (1H, m), 6.73 (1H, d, *J* = 7.8 Hz), 6.66 (1H, d, *J* = 5.1 Hz), 3.40 (2H, s), 2.81 (2H, t, *J* = 5.5 Hz), 2.68 (2H, t, *J* = 5.7 Hz), 2.38 (2H, t, *J* = 7.6 Hz), 2.32 (3H, s), 1.88 (2H, m), 1.75 (2H, m), 1.45 (2H, m), 1.10 (1H, m), 0.91 (1H, m), 0.61 (3H, t, *J* = 7.4 Hz) ppm.

¹³C-NMR (CDCl₃, TMS, 101 MHz): 182.31, 138.77, 133.79, 133.33, 132.64, 131.77, 127.88, 125.19, 123.78, 122.54, 109.08, 57.50, 54.18, 53.01, 50.79, 37.67, 31.10, 27.50, 25.35, 22.33, 21.21, 8.56 ppm.
**E**lementary analysis for the Formula C₂₂H₂₈N₂OS (368.55):
Calculated: C 71.70, H 7.66, N 7.60, S 8.70 %.
Found: C 71.19, H 7.61, N 7.42, S 8.55 %.

### Example 31

### 3-[4-(6,7-Dihydro-4H-thieno[3,2-c]pyridin-5-yl)-butyl]-3-ethyl-6-fluoro-1,3-dihydro-2H-indol-2-one monooxalate

The title compound is prepared according to process "D" by applying processing method 1 starting from 3-(4-chlorobutyl)-3-ethyl-6-fluoro-1,3-dihydro-2*H*-indol-2-one and 6,7-dihydro-4*H-*thieno[3,2-c]pyridine.
M.p.: 167-168 °C.

IR (KBr): 1707 (C=O), 1140 cm⁻¹.

¹H-NMR (DMSO-*d*₆, TMS, 400 MHz): 0.50 (3H, t, *J* = 7.3 Hz), 0.85-0.78 (1H, m), 0.99-0.85 (1H, m), 1.57-1.50 (2H, m), 1.79-1.67 (2H, m), 2.86 (2H, t, *J* = 7.4 Hz), 2.97 (2H, s), 3.2 (2H, s), 3.99 (2H, s), 5.0-4.2 (2H, br s), 6.66 (1H, dd, *J* = 2.3, 9.3 Hz), 6.78 (1H, dt, *J* = 2.3, 7.8 Hz), 6.84 (1H, d, *J* = 5.2 Hz), 7.2 (1H, dd, *J* = 5.7, 8.1 Hz), 7.40 (1H, d, *J* = 5.2 Hz), 10.5 (1H, s) ppm.

¹³C-NMR (DMSO-*d*₆, TMS, 50 MHz): 8.3, 21.4, 22.5, 24.5, 30.2, 36.5, 49.4, 50.8, 52.8, 55.1, 97.5 (d, *J* = 27.1 Hz), 107.5 (d, *J* = 22.5 Hz), 124.34 (d, *J* = 10.7 Hz), 124.6, 125.3, 127.8, 129.8, 131.8, 144.0 (d, *J* = 12.2 Hz), 161.9 (d, *J* = 240.7 Hz), 163.9, 181.0 ppm.

| Elementary analysis for the Formula C₂₃H₂₇FN₂O₅S (462.54): | | | | |
|---|---|---|---|---|
| Calculated: | C 59.73, | H 5.88, | N 6.06, | S 6.93 %. |
| Found: | C 59.80, | H 5.90, | N 6.01, | S 6.83 %. |

### Example 32

### 3-[4-(6,7-Dihydro-4H-thieno[3,2-c]pyridin-5-yl)-butyl]-3-ethyl-5-fluoro-1,3-dihydro-2H-indol-2-one monohydrochloride

The title compound is prepared according to process "D" starting from 3-(4-chlorobutyl)-3-ethyl-5-fluoro-1,3-dihydro-2*H*-indol-2-one and 6,7-dihydro-4*H*-thieno[3,2-c]pyridine. The product is isolated from the reaction mixture by applying processing method 2.
M.p.: 119-121 °C:

IR (KBr): 3441, 1712 (C=O), 1184 cm⁻¹.

¹H-NMR (CDCl₃, TMS, 400 MHz): 0.60 (1H, t, *J* = 7.4 Hz), 1.00-0.86 (1H, m), 1.16-1.04(1H, m), 1.95-1.66 (6H, m), 3.20-2.94 (2H, m), 3.22 (2H, br s), 3.50 (2H, br s), 4.33 (2H, br s), 6.78 (1H, d, *J* = 5.2 Hz), 6.81 (1H, dd, *J* = 2.5, 8.1 Hz), 6.87 (1H, dt, *J* = 2.5, 9.1 Hz), 6.95 (1H, dd, *J* = 4.5, 8.5 Hz), 7.21 (1H, d, *J* = 5.1 Hz), 9.74 (1H, s) ppm.

¹³C-NMR (CDCl₃, TMS, 101 MHz): 8.4, 21.1, 21.6, 24.0, 30.9, 36.6, 49.2, 50.3, 54.2, 54.3 (d, *J* = 1.9 Hz), 110.5 (d, *J* = 24.4 Hz), 110.7 (d, *J* = 8.0 Hz), 114.1 (d, *J* = 23.7 Hz), 124.7, 125.4, 126.3, 131.0, 133.5 (d, *J* = 7.6 Hz), 137.7, 159.0 (d, *J* = 239.9 Hz), 181.7 ppm.

| Elementary analysis for the Formula C₂₁H₂₆ClFN₂OS (408.97): | | | | | |
|---|---|---|---|---|---|
| Calculated: | C 61.68, | H 6.41, | Cl 8.67, | N 6.85, | S 7.84 %. |
| Found: | C 60.75, | H 6.43, | Cl 8.02, | N 6.73, | S 7.77 %. |

### Example 33

### 7-Chloro-3-[4-(6,7-dihydro-4H-thieno[3,2-c]-pyridin-5-yl)-butyl]-3-ethyl-5-fluoro-1,3-dihydro-2H-indol-2-one

The title compound is prepared according to process "D" starting from 7-chloro-3-(4-chlorobutyl)-3-ethyl-5-fluoro-1,3-dihydro-2*H-*indol-2-one and 6,7-dihydro-4*H*-thieno[3,2-c]-pyridine. The product is isolated from the reaction mixture by applying processing method 1.
M.p.: 194-196 °C (ethanol).

IR (KBr): 1726 (C=O) cm⁻¹.

¹H-NMR (DMSO-*d₆*, TMS, 400 MHz): 0.51 (3H, t, *J* = 7.4 Hz), 0.90-0.70 (1H, m), 1.08-0.90 (1H, m), 1.39-1.32 (2H, m), 1.82-1.70 (4H, m), 2.59 (2H, t, *J* = 5.6 Hz), 2.71 (2H, t, *J* = 5.2 Hz), 3.34 (2H, m), 6.74 (1H, d, *J* = 5.1 Hz), 7.25-7.22 (3H, m), 10.84 (1H, s) ppm.

¹³C-NMR (DMSO-*d*₆, TMS, 101 MHz): 8.5, 22.0, 25.1, 26.9,30.5, 37.0, 50.5, 52.6, 55.2 (d, *J* = 1.9 Hz), 56.8, 110.4 (d, *J* = 24.0 Hz), 113.4 (d, *J* = 11.1 Hz), 114.6 (d, *J* = 26.7 Hz), 123.0, 125.6, 132.9, 134.3, 135.8 (d, *J* = 8.8 Hz), 136.8 (d, *J* = 2.3 Hz), 158.0 (d, *J* = 240.3 Hz), 180.7 ppm.

| Elementary analysis for the Formula C₂₁H₂₄ClFN₂OS (406.95): | | | | | |
|---|---|---|---|---|---|
| Calculated: | C 61.98, | H 5.94, | Cl 8.71, | N 6.88, | S 7.88 %. |
| Found: | C 61.66, | H 5.92, | Cl 8.52, | N 6.84, | S 7.86 %. |

### Example 34

### 3-[4-(2-Chloro-6,7-dihydro-4H-thieno[3,2-c]-pyridin-5-yl)-butyl]-3-ethyl-1,3-dihydro-2H-indol-2-one

The title compound is prepared according to process "D" starting from 3-(4-chlorobutyl)-3-ethyl-1,3-dihydro-2*H*-indol-2-one and 2-chloro-6,7-dihydro-4*H*-thieno[3,2-c]pyridine. The product is isolated from the reaction mixture by applying processing method 1.
M.p.: 117-119 °C (hexane-ethyl acetate).

IR (KBr): 3299, 1705 (C=O), 769 cm⁻¹.

¹H-NMR (CDCl₃, TMS, 400 MHz): 0.62 (3H, t, *J* = 7.4 Hz), 0,95-0.89 (1H, m), 1.18-1.08 (1H, m), 1.51-1.39 (2H, m), 1.84-1.74 (2H, m), 1.97-1.87 (2H, m), 2.36 (2H, t, *J* = 7.8 Hz), 2.70-2.64 (4H, m), 3.34 (2H, s), 6.49 (1H, s), 6.89 (1H, d, *J* = 7.8 Hz), 7.06 (1H, dt, *J* = 1.0, 7.5 Hz), 7.10 (1H, d, *J* = 6.4 Hz), 7.20 (1H, dt, *J* = 1.3, 7.6 Hz), 8.81 (1H, br s) ppm.

¹³C-NMR (CDCl₃, TMS, 101 MHz): 182.6, 141.4, 133.1, 132.5, 132.1, 127.6, 127.0, 124.2, 123.0, 122.3, 109.5, 57.3, 54.2, 52.5, 50.4, 37.5, 31.0, 27.3, 25.1, 22.2, 8.5 ppm.

| Elementary analysis for the Formula C₂₁H₂₅ClN₂OS (388.96): | | | | | |
|---|---|---|---|---|---|
| Calculated: | C 64.85, | H 6.48, | Cl 9.11, | N 7.20, | S 8.24 %. |
| Found: | C 65.14, | H 6.33, | Cl 9.00, | N 7.01, | S 8.01 %. |

### Example 35

### 5-Chloro-3-[4-(6,7-dihydro-4H-thieno[3,2-c]-pyridin-5-yl)-butyl]-3-ethyl-6-fluoro-1,3-dihydro-2H-indol-2-one

The title compound is prepared according to process "D" starting from 5-chloro-3-(4-chlorobutyl)-3-ethyl-6-fluoro-1,3-dihydro-2*H-*indol-2-one and 6,7-dihydro-4*H*-thieno[3,2-c]-pyridine. The product is isolated from the reaction mixture by applying processing method 1.
M.p.: 155-157 °C (ethanol).

IR (KBr): 3112, 1722 (C=O), 1160, 727 cm⁻¹.

¹H-NMR (CDCl₃, TMS, 400MHz): 0.63 (3H, t, *J* = 7.4 Hz), 0.96-0.90 (1H, m), 1.18-1.05 (1H, m), 1.55-1.43 (2H, m), 1.80-1.70 (2H, m), 1.96-1.86 (2H, m), 2.42 (2H, t, *J* = 7.7 Hz), 2.72 (2H, t, *J* = 5.2 Hz), 2.83 (2H, t, *J* = 5.3 Hz), 3.48 (2H, s), 6.67 (1H, d, *J* = 8.8 Hz), 6.69 (1H, *J* = 5.1 Hz), 7.05 (1H, *J* = 5.1 Hz), 7.10 (1H, d, *J* = 7.1 Hz), 8.88 (1H, s) ppm.

¹³C-NMR (CDCl₃, TMS, 101 MHz): 8.5, 22.2, 25.3, 27.4, 31.1, 37.6, 50.8, 53.0, 54.2, 57.3, 99.2 (d, *J* = 26.7 Hz), 114.1 (d, *J* = 18.7 Hz), 122.7, 124.8, 125.2, 129.0 (d, *J* = 3.8 Hz), 133.3, 133.7, 141.0 (d, *J* = 10.7 Hz), 157.5 (d, *J* = 247.2 Hz), 182.1 ppm.

| Elementary analysis for the Formula C₂₁H₂₄ClFN₂OS (406.95): | | | | | |
|---|---|---|---|---|---|
| Calculated: | C 61.98, | H 5.94, | Cl 8.71, | N 6.88, | S 7.88 %. |
| Found: | C 60.52, | H 5.65, | Cl 9.17, | N 6.57, | S 7.68 %. |

### Example 36

### 3-[5-(6,7-Dihydro-4H-thieno[3,2-c]pyridin-5-yl)-pentyl]-3-ethyl-1,3-dihydro-2H-indol-2-one monooxalate

The title compound is prepared according to process "D" starting from 3-(5-bromopentyl)-3-ethyl-1,3-dihydro-2*H*-indol-2-one and 6,7-dihydro-4*H*-thieno[3,2-c]pyridine. The product is isolated from the reaction mixture by applying processing method 3.
M.p.: 193-195 °C.

IR (KBr): 3200-3100, 1763, 1710 cm⁻¹.

¹H-NMR (DMSO-*d*₆, TMS, 400 MHz): 0.50 (3H, t, *J* = 7.4 Hz), 0.83-0.78 (1H, m), 1.00-0.95 (1H, m), 1.20-1.12 (2H, m), 1.69-1.52 (4H, m), 2.95 (2H, t, *J* = 8.1 Hz), 3.03 (2H, t, *J* = 5.6 Hz), 3.36 (2H, t, *J* = 5.7 Hz), 4.14 (2H, s), 6.85 (1H, d, *J* = 7.5 Hz), 6.86 (1H, d, *J* = 5.2 Hz), 6.98 (1H, dt, *J* = 0.9, 7.5 Hz), 7.16 (1H, dt, *J* = 1.2, 7.7 Hz), 7.19 (1H, d, *J* = 7.3 Hz), 9.4-8.4 (2H, br s), 10.37 (1H, s) ppm.

¹³C-NMR (DMSO-*d₆*, TMS, 101 MHz): 180.9, 164.4, 142.7, 132.4, 131.7, 129.2, 127.7, 125.4, 125.0, 123.1, 121.6, 109.3, 55.0, 53.2, 50.5, 49.3, 36.9, 30.5, 26.3, 23.8, 23.8, 22.2, 8.6 ppm.

| Elementary analysis for the Formula C₂₄H₃₀N₂O₅S (458.58): | | | | |
|---|---|---|---|---|
| Calculated: | C 62.86, | H 6.59, | N 6.11, | S 6.99 %. |
| Found: | C62.43, | H 6.58, | N 6.10, | S 6.83 %. |

### Example 37

### 3-[5-(2-Chloro-6,7-dihydro-4H-thieno[3,2-c]-pyridin-5-yl)-pentyl]-3-ethyl-1,3-dihydro-2H-indol-2-one monohydrochloride

The title compound is prepared according to process "D" starting from 3-(5-bromopentyl)-3-ethyl-1,3-dihydro-2*H*-indol-2-one and 2-chloro-6,7-dihydro-4*H*-thieno[3,2-c]pyridine. The product is isolated by applying processing method 2.
M.p.: 96-98 °C.

IR (KBr): 3426, 2549, 1708 (C=O) cm⁻¹.
¹H-NMR (CDCl₃, TMS, 400 MHz): 0.61 (3H, t, *J* = 7.4 Hz), 0.96-0.86 (1H, m), 1.10-1.04 (1H, m), 1.29-1.20 (2H, m), 1.90-1.70 (6H, m), 3.10 (2H, t, *J* = 8.2 Hz), 3.6-3.2 (4H, br s), 4.5-3.8 (2H, br s), 6.62 (2H, s), 6.95 (1H, d, *J* = 7.7 Hz), 7.03 (1H, dt, *J* = 0.9, 7.4 Hz), 7.09 (1H, d, *J* = 6.4 Hz), 7.19 (1H, dt, *J* = 1.4, 7.5 Hz), 8.94 (1H, s) ppm.

¹³C-NMR (CDCl₃, TMS, 101 MHz): 182.2, 141.4, 132.2, 130.3, 129.9, 127.7, 125.8, 123.7, 122.9, 122.4, 109.8, 54.9, 54.0, 49.8, 49.0, 37.0, 31.1, 26.6, 23.7, 23.6, 21.2, 8.5 ppm.

| Elementary analysis for the Formula C₂₂H₂₈Cl₂N₂OS (439.45): | | | | | |
|---|---|---|---|---|---|
| Calculated: | C 60.13, | H 6.42, | Cl 16.14, | N 6.37, | S 7.30 %. |
| Found: | C 59.59, | H 6.35, | Cl 15.82, | N 6.23, | S 7.05 %. |

### Example 38

### 3-[4-(3,4-Dihydro-1H-isoquinolin-2-yl)-butyl]-3-ethyl-1,3-dihydro-2H-indol-2-one monohydrochloride

The title compound is prepared according to process "D" starting from 3-(4-chlorobutyl)-3-ethyl-1,3-dihydro-2*H*-indol-2-one and 3,4-dihydro-1*H*-isoquinoline. The product is isolated from the reaction mixture by applying processing method 2.
M.p.: 113-115 °C.

IR (KBr): 3420, 2875, 1709 (C=O) cm⁻¹.

¹H-NMR (DMSO-*d*₆, TMS, 400 MHz): 0.47 (3H, t, *J* = 7.3 Hz), 0.83-0.80 (1H, m), 1.00-0.95 (1H, m), 1.76-1.65 (6H, m), 2.98-2.89 (3H, m), 3.18-3.15 (2H, m), 3.54 (1H, br s), 4.10 (1H, m), 4.15 (1H, d, *J* = 4.7 Hz), 6.83 (1H, d, *J* = 7.6 Hz), 6.96 (1H, dt, *J* = 0.9, 7.5 Hz), 7.24-7.12 (6H, m), 10.4 (1H, s), 11.1 (1H, br s) ppm.

¹³C-NMR (DMSO-*d*₆, TMS, 101 MHz): 8.6, 21.5, 23.5, 24.9, 30.4, 36.6, 48.7, 51.5, 53.2, 54.9, 109.4, 121.7, 123.2, 126.7, 126.7, 127.7, 127.8, 128.6, 128.7, 131.6, 132.1, 142.7,180.8 ppm.

| Elementary analysis for the Formula C₂₃H₂₉ClN₂O (384.95): | | | | |
|---|---|---|---|---|
| Calculated: | C 71.76, | H 7.59, | Cl 9.21, | N 7.28 %. |
| Found: | C 69.76, | H 7.78, | Cl 8.75, | N 6.99 %. |

### Example 39

### 3-Ethyl-3-{4-[4-(4-fluorophenyl)-1,2,3,6-tetrahydropyridin-1-yl]-butyl}-1,3-dihydro-2H-indol-2-one monohydrochloride

The title compound is prepared according to process "D" starting from 3-(4-chlorobutyl)-3-ethyl-1,3-dihydro-2*H*-indol-2-one and 4-(4-fluoro-phenyl)-1,2,3,6-tetrahydropyridine. The product is isolated from the reaction mixture by applying processing method 2.
M.p.: 108-111 °C.

IR (KBr): 3426, 1705 (C=O) cm⁻¹.

¹H-NMR (DMSO-*d*₆, TMS, 400 MHz): 0.51 (3H, t, *J* = 7.4 Hz), 0.78-0.88 (1H, m), 0.94-1.02 (1H, m), 1.64-1.83 (6H, m), 2.86-3.80 (6H, m), 2.98 (2H, t, *J* = 8.1 Hz), 6.12 (1H, s), 6.87 (1H, d, *J* = 7.7 Hz), 7.00 (1H, dt, *J* = 0.9, 7.5 Hz), 7.15-7.23 (4H, m), 7.52 (2H, m), 10.45 (1H, s), 10.9 (1H, br s) ppm.

¹³C-NMR (DMSO-*d*₆, TMS, 400 MHz): 8.6, 21.5, 23.6, 23.8, 30.4, 36.6, 48.0, 49.4, 53.2, 54.6, 109.3, 115.5 (d, *J* = 21.4 Hz), 116.5, 121.7, 123.2, 127.0 (d, *J* = 8.0 Hz), 127.8, 132.1, 133.3, 134.9 (d, *J* = 3.1 Hz), 142.7, 162.0 (d, *J* = 244.9 Hz), 180.8 ppm.

| Elementary analysis for the Formula C₂₅H₃₀ClFN₂O (428.98): | | | | |
|---|---|---|---|---|
| Calculated: | C 70.00, | H 7.05, | Cl 8.26, | N 6.53 %. |
| Found: | C 66.90, | H 6.60, | Cl 7.67, | N 6.22 %. |

### Example 40

### 3-{4-[4-(4-Chlorophenyl)-3,6-dihydro-2H-pyridin-1-yl]-butyl}-3-ethyl-1,3-dihydro-2H-indol-2-one

The title compound is prepared according to process "D" starting from 3-(4-chlorobutyl)-3-ethyl-1,3-dihydro-2*H*-indol-2-one and 4-(4-chlorophenyl)-1,2,3,6-tetrahydropyridine. The product is isolated from the reaction mixture by applying processing method 1.
M.p.: 142-145 °C (hexane-ethyl acetate).

IR (KBr): 3181, 1715, 1701 (C=O) cm⁻¹.

¹H-NMR (DMSO-*d*₆, TMS, 400 MHz): 0.61 (3H, t, *J* = 7.4 Hz), 0.96-0.84 (1H, m), 1.17-1.04 (1H, m), 1.46-1.35 (2H, m), 1.95-1.73 (4H, m), 2.29 (2H, t, *J* = 7.8 Hz), 2.45 (2H, m), 2.58 (2H, t, *J* = 5.6 Hz), 3.03 (2H, q, *J* = 2.8 Hz), 5.99 (1H, t, *J*= 1.8 Hz), 6.88 (1H, d, *J* = 7.7 Hz), 7.02 (1H, dt, *J* = 1.0, 7.5 Hz), 7.10 (1H, d, *J* = 6.4 Hz), 7.17 (1H, dt, *J* = 1.4, 7.6 Hz), 7.27-7.21 (4H, m), 8.63 (1H, s) ppm.

¹³C-NMR (DMSO-*d*₆, TMS, 101 MHz): 8.6, 22.1, 26.9, 27.4, 30.5, 37.2, 49.9, 52.8, 53.3, 57.5, 109.2, 121.6, 123.1, 123.1, 126.4, 127.6, 128.4, 131.5, 132.4, 132.9, 139.1, 142.7, 181.0 ppm.

| Elementary analysis for the Formula C₂₅H₂₉ClN₂O (408.98): | | | | |
|---|---|---|---|---|
| Calculated: | C 73.42, | H 7.15, | Cl 8.67, | N 6.85 %. |
| Found: | C 71.98, | H 7.07, | Cl 8.41, | N 7.09 %. |

### Example 41

### 3-{4-[4-(3-Chlorophenyl)-3,6-dihydro-2H-pyridin-1-yl]-butyl}-3-ethyl-1,3-dihydro-2H-indol-2-one monohydrochloride

The title compound is prepared according to process "D" starting from 3-(4-chlorobutyl)-3-ethyl-1,3-dihydro-2*H*-indol-2-one and 4-(3-chlorophenyl)-1,2,3,6-tetrahydropyridine. The product is isolated from the reaction mixture by applying processing method 2.
M.p.: 82-85 °C.

IR (KBr): 3421, 3168, 1706 (C=O) cm⁻¹.
¹H-NMR (DMSO-*d*₆, TMS, 200 MHz): 0.51 (3H, t, *J* = 7.3 Hz), 1.03-0.83 (2H, m), 1.95-1.60 (6H, m), 4.0-2.76 (8H, m), 6.25 (1H, s), 6.87 (1H, d, *J* = 7.6 Hz), 7.00 (1H, t, *J* = 7.3 Hz), 7.52-7.15 (6H, m), 10.47 (1H, s), 10.92 (1H, br s) ppm.

¹³C-NMR (DMSO-*d*₆, TMS, 50.3 MHz): 8.62, 21.5, 23.6, 30.4, 36.7, 47.9, 49.4, 53.2, 54.6, 109.4, 118.2, 121.7, 123.2, 123.7, 124.9, 127.8, 127.9, 130.6, 132.2, 133.1, 133.7, 140.7, 142.7, 180.8 ppm.

| Elementary analysis for the Formula C₂₅H₃₀Cl₂N₂O (445.44): | | | | |
|---|---|---|---|---|
| Calculated: | C 67.41, | H 6.79, | Cl 15.92, | N 6.29 %. |
| Found: | C 65.14, | H 6.64, | Cl 15.26, | N 6.02 %. |

### Example 42

### 3-[6-(2-chloro-6,7-dihydro-4H-thieno[3,2-c]pyridine-5-yl)-hexyl]-3-ethyl-1,3-dihydro-2H-indole-2-one monohydrochloride

The title compound is prepared according to Process "D" starting from 3-(6-bromohexyl)-3-ethyl-1,3-dihydro-2*H*-indole-2-one and 2-chloro-6,7-dihydro-4*H*-thieno[3,2-c]pyridine. The product is isolated according to workup Procedure 2 from the reaction mixture.

Melting point, 82-85 °C.

IR (KBr): 3169,2560,1708 (C=O), 752 (C-Cl) cm⁻¹.

¹H-NMR (DMSO-*d*₆, TMS, 200 MHz): 0.49 (3H,, *J* = 7.3 Hz), 0.95-0.78 (2H, m), 1.24-1.15 (4H, m), 1.74 -1.65 (6H, m), 3.05 (4H, t, *J* = 7.3 Hz), 3.38 (2H, m), 4.14 (2H, m), 6.85 (1H, d, *J* = 7.7 Hz), 6.94 (1H, s), 7.01 (1H, dt, *J* = 1.1, 8.4 Hz), 7.21-7.12 (3H, m), 10.42 (1H, s), 11.3 (1H, sz) ppm.

¹³C-NMR (DMSO-*d*₆, TMS, 50.3 MHz): 8.4, 1.7, 23.3, 23.7, 25.9, 28.7, 30.3, 36.9, 48.6, 49.3, 53.1, 54.7, 109.1, 121.4, 122.9, 124.8, 127.0, 127.5, 128.1, 131.0, 132.2, 142.5, 180.8 ppm.

| Elemental Analysis for the Formula C₂₃H₃₀Cl₂N₂OS (453.48) | | | | | |
|---|---|---|---|---|---|
| Calculated: | C 60.92, | H 6.67, | Cl 15.64, | N 6.18, | S 7.07 %. |
| Found: | C 60.48, | H 6.85, | Cl 15.08, | N 6.20, | S 6.84 %. |

### Example 43

### 3-{4-[4-(3-chlorophenyl)-3,6-dihydro-2H-pyridine-1-yl]-butyl}-3-ethyl-5-fluoro-1,3-dihydro-2H-indol-2-one

The title compound is prepared according to process D starting from 3-(4-chlorobutyl)-3-ethyl-5-fluoro-1,3-dihydro-2*H*-indol-2-one and 4-(3-chlorophenyl)-1,2,3,6-tetrahydro-pyridine. The product is isolated by processing method 1.

Melting point, 112-114 °C.

IR (KBr): 3161, 1706 (C=O), 817 cm⁻¹.

¹H-NMR (DMSO-*d*₆, TMS, 500 MHz): 0.50 (3H, t, *J* = 7.4 Hz), 0.81-0.77 (1H, m), 0.98-0.94 (1H, m), 1.38-1.28 (2H, m), 1.80-1.68 (4H, m), 2.23 (2H, t, *J* = 7.2 Hz), 2.38 (2H, d, *J* = 1.6 Hz), 2.52-2.48 (2H, m), 2.96 (2H, t, *J* = 2.6 Hz), 6.19 (1H, kv, *J* = 1.8 Hz), 6.80 (1H, dd, *J* = 4.5, 8.2 Hz), 6.98 (1H, ddd, *J* = 2.7, 8.5, 9.8 Hz), 7.16 (1H, dd, *J* = 2.7, 8.5 Hz), 7.28 (1H, td, *J* = 1.8, 7.4 Hz), 7.33 (1H, t, *J* = 7.7 Hz), 7.37 (1H, td, *J* = 1.6, 7.9 Hz), 7.42 (1H, t, *J* = 1.6 Hz), 10.35 (1H, s) ppm.

¹³C-NMR (DMSO-*d*₆, TMS, 125.6 MHz): 8.5, 22.0, 26.8, 27.3, 30.4, 37.0, 49.8, 52.8, 54.1, 57.4, 109.8 (d, *J* = 7.8 Hz), 111.2 (d, *J* = 24.4 Hz), 113.8 (d, *J* = 23.4 Hz), 123.3, 123.9, 124.5, 126.8, 130.3, 132.9, 133.5, 134.5 (d, *J* = 7.8 Hz), 138.8, 142.5, 158.3 (d, *J* = 236.3 Hz), 180.9 ppm.

| Elemental analysis for the Formula C₂₅H₂₈ClFN₂O (426.97) | | | | |
|---|---|---|---|---|
| Calculated: | C 70.33, | H 6.61, | Cl 8.30, | N 6.56 %. |
| Found: | C 70.74, | H 6.44, | Cl 8.37, | N 6.68 %. |

### Example 44

### 3-{4-[4-(4-chlorophenyl)-3,6-dihydro-2H-pyridin-1-yl]-butyl}-3-ethyl-5-fluoro-1,3-dihydro-2H-indol-2-one

The title compound is prepared according to process D starting from 3-(4-chlorobutyl)-3-ethyl-5-fluoro-1,3-dihydro-2*H*-indol-2-one and 4-(4-chlorophenyl)-1,2,3,6-tetrahydro-pyridine. The product is isolated using processing method 1.

Melting point, 146-148 °C.

IR (KBr): 3289, 1717 (C=O), 1689, 818 cm⁻¹.

¹H-NMR (DMSO-*d*₆, TMS, 500 MHz): 0.50 (3H, t, *J* = 7.4 Hz), 0.83-0.77 (1H, m), 0.99-0.93 (1H, m), 1.40-1.31 (2H, m), 1.51-1.68 (4H, m), 2.28 (2Hm sz), 2.40 (2H, sz), 2.56 (2H, sz), 3.02 (2H, sz), 6.14 (1H, kv, *J*= 1.9 Hz), 6.81 (1H, dd, *J* = 4.5, 8.4 Hz), 6.98 (1H, ddd, *J* = 2.7, 8.4, 9.7 Hz), 7.16 (1H, dd, *J* = 2.8, 8.4 Hz), 7.37 (2H, d, *J* = 8.8 Hz), 7.43 (2H, d, *J* = 8.8 Hz), 10.38 (1H, s) ppm.

¹³C-NMR (DMSO-*d*₆, TMS, 125.6 MHz): 8.5, 22.0, 26.5, 27.1, 30.4, 37.0, 49.7, 52.5, 54.1 (d, *J* = 2.0 Hz), 57.2, 109.8 (d, *J* = 7.8 Hz), 111.1 (d, *J* = 23.9 Hz), 113.8 (d, *J* = 23.0 Hz), 122.6, 126.4, 128.4, 131.6, 132.9, 134.5 (d, *J* = 7.8 Hz), 138.8 (d, *J* = 1.5 Hz), 138.9, 158.3 (d, *J* = 236.3 Hz), 180.8 ppm.

| Elemental analysis for the Formula C₂₅H₂₈ClFN₂O (426.97) | | | | |
|---|---|---|---|---|
| Calculated: | C 70.33, | H 6.61, | Cl 8.30, | N 6.56 %. |
| Found: | C 69.03, | H 6.95, | Cl 8.66, | N 6.28 %. |

### Example 45

### 3-{4-[4-(3-chlorophenyl)-3,6-dihydro-2H-pyridin-1-yl]-butyl}-3-etyl-6-fluoro-1,3-dihydro-2H-indol-2-one monohydrochloride

The title compound is prepared according to process D using 3-(4-chlorobutyl)-3-ethyl-6-fluoro-1,3-dihydro-2*H*-indol-2-one and 4-(3-chlorophenyl)-1,2,3,6-tetrahydro-pyridine as starting compound. The product is isolated according to processing method 2.

Melting point, 101-104 °C.

IR (KBr): 3158, 2877, 1715 (C=O) cm⁻¹.

¹H-NMR (DMSO-*d*₆, TMS, 500 MHz): 0.51 (3H, t, *J* = 7.4 Hz), 0.97-0.80 (2H, m), 1.81-1.63 (6H, m), 3.73-2.51 (8H, m), 6.20 (1H, s), 6.82-6.67 (2H, m), 7.50-7.20 (5H, m), 8.8 (1H, sz), 10.6 (1H, s) ppm.

| Elemental analysis for the Formula C₂₅H₂₉Cl₂FN₂O (463.43) | | | | |
|---|---|---|---|---|
| Calculated: | C 64.80, | H 6.31, | Cl 15.30, | N 6.04 %. |
| Found: | C 64.74, | H 6.51, | Cl 15.55, | N 6.26 %. |

## Claims

1. 3,3-Disubstituted indol-2-one derivatives of the general Formula (I), 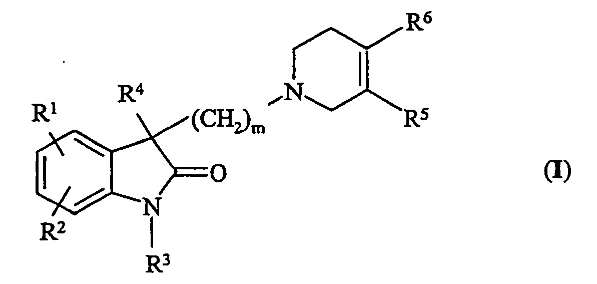 wherein
R¹ and R² independently represent hydrogen, halogen, alkyl having 1 to 7 carbon atom(s) or sulfamoyl;
R³ represents hydrogen or straight or branched chain alkyl having 1 to 7 carbon atom(s);
R⁴ stands for alkyl having 1 to 7 carbon atom(s);
R⁵ is hydrogen and R⁶ denotes phenyl optionally carrying 1 to 3 substituent(s) selected from halogen and alkyl having 1 to 7 carbon atom(s) carrying 1 to 3 halogen substituent(s), or
R⁵ and R⁶ form, together with the adjacent carbon atoms of the tetrahydropyridine ring, a phenyl group or a 5- or 6-membered heterocyclic ring containing a sulfur as heteroatom, which may optionally carry a halogen substituent;
m is 1, 2, 3, 4, 5 or 6,
and pharmaceutically acceptable acid addition salts thereof.

2. 3,3-Disubstituted indol-2-one derivatives of the general Formula (I) according to claim 1 wherein
R¹, R² and R³ independently represent hydrogen, halogen or straight or branched chain alkyl having 1 to 7 carbon atom(s), R⁴ is ethyl, R⁵ denotes hydrogen and R⁶ stands for phenyl optionally carrying a halogen or a trifluoromethyl substituent, or
R⁵ and R⁶ form, together with the adjacent carbon atoms of the tetrahydropyridine ring, phenyl or a 5- or 6-membered heterocyclic ring containing a sulfur atom as heteroatom, which optionally carries a halogen atom, m is 3, 4 or 5, and pharmaceutically acceptable acid addition salts thereof.

3. The following compounds of the general Formula (I) according to claim 1:
3-ethyl-3-{4-[4-(3-trifluoromethyl-phenyl)-1,2,3,6-tetrahydropyridin-1-yl]-butyl}-1,3-dihydro-2*H*-indol-2-one,
3-[4-(6,7-dihydro-4*H*-thieno[3,2-c]pyridin-5-yl)-butyl]-3-ethyl-1,3-dihydro-2*H*-indol-2-one,
3-[5-(6,7-dihydro-4*H*-thieno[3,2-c]pyridin-5-yl)-pentyl]-3-ethyl-1,3-dihydro-2*H*-indol-2-one,
3-[5-(2-chloro-6,7-dihydro-4*H*-thieno[3,2-c]pyridin-5-yl)-pentyl]-3-ethyl-1,3-dihydro-2*H*- indol-2-one,
3-[4-(3,4-dihydro-1*H*-isoquinolin-2-yl)-butyl]-3-ethyl-1,3-dihydro-2*H*-indol-2-one,
3-ethyl-3-{4-[4-(4-fluorophenyl)-1,2,3,6-tetrahydropyridin-1-yl]-butyl}-1,3-dihydro-2*H*-indol-2-one,
3-ethyl-3-{4-[4-(4-fluorophenyl)-1,2,3,6-tetrahydropyridin-1-yl]-butyl}-1,3-dihydro-2*H*-indol-2-one,
3-{4-[4-(4-chlorophenyl)-3,6-dihydro-2*H*-pyridin-1-yl]-butyl}-3-ethyl-1,3-dihydro-2*H*-indol-2-one,
3-{4-[4-(3-chlorophenyl)-3,6-dihydro-2*H*-pyridin-1-yl]-butyl}-3-ethyl-1,3-dihydro-2*H*-indol-2-one,
and pharmaceutically acceptable acid addition salts thereof.

4. 3-[5-(6,7-Dihydro-4*H*-thieno[3,2-c]pyridin-5-yl)-pentyl]-3-ethyl-1,3-dihydro-2*H*-indol-2-one according to claim 1 and pharmaceutically acceptable acid addition salts thereof.

5. 3-Ethyl-3- {4-[4-(4-fluorophenyl)-1,2,3,6-tetrahydropyridin-1-yl]-butyl}-1,3-dihydro-2*H*-indol-2-one according to claim 1, and pharmaceutically acceptable acid addition salts thereof.

6. Pharmaceutical compositions comprising as active ingredient at least one of the compounds of the general Formula (I) or a pharmaceutically acceptable acid addition salt thereof in admixture with one or more conventional carrier(s) or auxiliary agent(s).

7. Pharmaceutical compositions according to claim 6 useful for the treatment or prophylaxis of central nervous disorders, particularly depression, anxiety, schizophrenia, mood disorders, mania, mental decline, stroke, cell death in certain areas of the central nervous system, stress disease, gastrointestinal diseases or cardiovascular diseases.

8. Pharmaceutical compositions according to claim 6 or 7 comprising as active ingredient 3-[5-(6,7-dihydro-4*H*-thieno[3,2-c]pyridin-5-yl)-pentyl]-3-ethyl-1,3-dihydro-2*H*-indol-2-one or 3-ethyl-3-{4-[4-(4-fluorophenyl)-1,2,3,6-tetrahydropyridin-1-yl]-butyl}-1,3-dihydro-2*H*-indol-2-one or pharma-ceutically acceptable acid addition salts thereof in admixture with one or more conventional carrier(s) or auxiliary agent(s).

9. A process for the preparation of a compound of the general Formula (I), wherein
R¹ and R² independently represent hydrogen, halogen, alkyl having 1 to 7 carbon atom(s) or sulfamoyl;
R³ represents hydrogen or straight or branched chain alkyl having 1 to 7 carbon atom(s);
R⁴ stands for alkyl having 1 to 7 carbon atom(s);
R⁵ is hydrogen and R⁶ denotes phenyl optionally carrying 1 to 3 substituent(s) selected from halogen and alkyl having 1 to 7 carbon atom(s) carrying 1 to 3 halogen substituent(s), or
R⁵ and R⁶ form, together with the adjacent carbon atoms of the tetrahydropyridine ring, a phenyl group or a 5- or 6-membered heterocyclic ring containing a sulfur as heteroatom, which may optionally carry a halogen substituent;
m is 1, 2, 3, 4, 5 or 6,
which comprises
(a) reacting a compound of the general Formula (II), 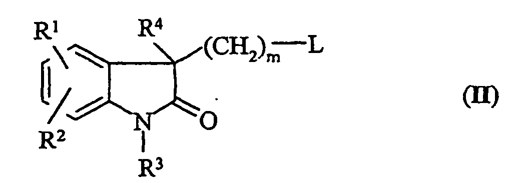 wherein R¹-R⁴ are as stated above and L is a leaving group, preferably chlorine or bromine, m is 1, 2, 3, 4, 5 or 6,, with a piperidine derivative of the general Formula (III), 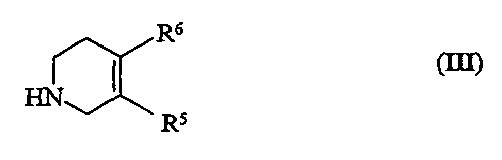 wherein R⁵ and R⁶ are as stated above, or
(b) reacting a compound of the general Formula (IV), 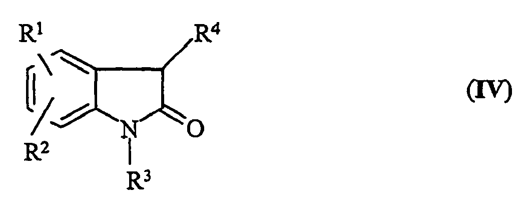 wherein R¹, R², R³ and R⁴ are as stated above, with a compound of the general Formula (V),
L-(CH₂)ₘ-L' (V)
wherein m is 1, 2, 3, 4, 5 or 6,, L and L' represent a leaving group, preferably chlorine or bromine, in the presence of a strong base, optionally halogenating the thus-obtained compound of the general Formula (II), wherein R² is hydrogen, and reacting the thus-obtained compound of the general Formula (II), wherein L is a leaving group, preferably chlorine or bromine, R² is hydrogen or halogen and m is 1, 2, 3, 4, 5 or 6,, with a pyridine derivative of the general Formula (III), wherein R⁵ and R⁶ are as stated above, in the presence of an acid binding agent, or
(c) reacting a compound of the general Formula (IV), wherein R¹, R², R³ and R⁴ are as stated above, with a pyridine derivative of the general Formula (VI), 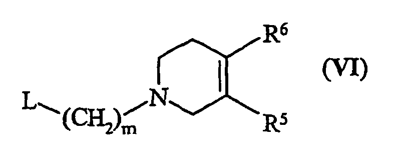 wherein L is sulfonyloxy or halogen, preferably chlorine or bromine; R⁵ and R⁶ are as stated above; m is 1, 2, 3, 4, 5 or 6,, in the presence of a strong base, and optionally halogenating the thus-obtained product, wherein R² is hydrogen, or liberating the free base from a salt thereof or converting it into a pharmaceutically acceptable, organic or inorganic acid addition salts thereof.

10. Compounds of the general Formula (I) according to any of claims 1 - 5 for use as a medicament.

11. A process for the preparation of pharmaceutical compositions useful for the treatment or prophylaxis of central nervous disorders, particularly depression, anxiety, schizophrenia, mood disorders, mania, mental decline, stroke, cell death in certain areas of the central nervous system, stress disease, gastrointestinal diseases or cardiovascular diseases, which comprises admixing at least one compound of the general Formula (I) or a pharmaceutically acceptable acid addition salts thereof according to any of claims 1 to 5 or pharmaceutically acceptable acid addition salts thereof with a pharmaceutical carrier and optionally other auxiliary agent and bringing the mixture to galenic form.

12. Use of a pharmaceutical composition containing at least one compound of the general Formula (I) or a pharmaceutically acceptable, organic or inorganic acid addition salt thereof in the preparation of a medicament for the treatment or prophylaxis of central nervous system disorders, particularly depression, anxiety, schizophrenia, mood disorder, mania, mental decline, stroke, cell death in certain areas of the central nervous system, stress disease, gastrointestinal diseases, cardiovascular diseases.

13. A process according to variants (a)-(c) of claim 9, which comprises carrying out the reaction in an apolar, dipolar aprotic or polar protic solvent, such as an aliphatic hydrocarbon or halogenated aliphatic hydrocarbon, or in a solvent of aromatic hydrocarbon, ether, ester, nitrile or ketone type or in a straight or branched chain alcohol having 1 to 4 carbon atom(s) or mixtures of such solvents.

14. A process according to any of variants (a) - (c) of claim 9, which comprises carrying out the reaction in the melt.

## Patentansprüche

1. 3,3-Disubstituierte Indol-2-on-Derivate der allgemeinen Formel (I) 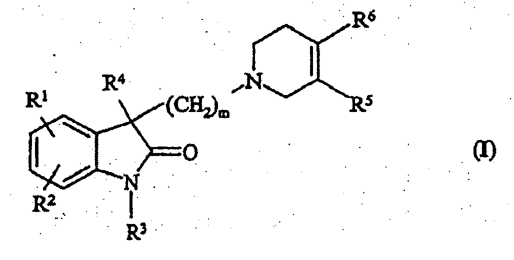 worin
R¹ und R² unabhängig Wasserstoff, Halogen, Alkyl mit 1 bis 7 Kohlenstoffatom(en) oder Sulfamoyl bedeuten;
R³ Wasserstoff oder gerad- oder verzweigtkettiges Alkyl mit 1 bis 7 Kohlenstoffatom(en) bedeuten;
R⁴ für Alkyl mit 1 bis 7 Kohlenstoffatom(en) steht;
R⁵ Wasserstoff ist und R⁶ Phenyl bedeutet, das gegebenenfalls 1 bis 3 Substituent(en) trägt, die aus Halogen und Alkyl mit 1 bis 7 Kohlenstoffatom(en), 1 bis 3 Halogensubstituent(en) tragend, gewählt wird, oder
R⁵ und R⁶ zusammen mit den benachbarten Kohlenstoffatomen des Tetrahydropyridinrings eine Phenylgruppe oder einen 5- oder 6-gliedrigen heterocyclischen Ring, der ein Schwefelatom als Heteroatom enthält, bilden können, welcher gegebenenfalls einen Halogensubstituenten tragen kann;
m 1, 2, 3, 4, 5 oder 6 ist,
und pharmazeutisch annehmbare Säureadditionssalze davon.

2. 3,3-Disubstituierte Indol-2-on-Derivate der allgemeinen Formel (I) gemäß Anspruch 1, worin
R¹, R² und R³ unabhängig Wasserstoff, Halogen oder gerad- oder verzweigtkettiges Alkyl mit 1 bis 7 Kohlenstoffatomen bedeuten, R⁴ Ethyl ist, R⁵ Wasserstoff bedeutet und R⁶ für Phenyl steht, das gegebenenfalls ein Halogen oder einen Trifluormethylsubstituenten trägt, oder
R⁵ und R⁶ zusammen mit den benachbarten Kohlenstoffatomen von dem Tetrahydropyridinring Phenyl oder einen 5- oder 6-gliedrigen heterocyclischen Ring bilden, welcher ein Schwefelatom als Heteroatom enthält, welcher gegebenenfalls ein Halogenatom trägt, m 3, 4 oder 5 ist, und pharmazeutisch annehmbare Säureadditionssalze davon.

3. Die folgenden Verbindungen der allgemeinen Formel (I) gemäß Anspruch 1:
3-Ethyl-3-{4-[4-(3-trifluormethyl-phenyl)-1,2,3,6-tetrahydropyridin-1-yl]-butyl}-1,3-dihydro-2*H*-indol-2-on,
3-[4-(6,7-Dihydro-4*H*-thieno[3,2-c]pyridin-5-yl)-butyl]-3-ethyl-1,3-dihydro-2*H*-indol-2-on,
3-[5-(6,7-Dihydro-4*H*-thieno[3,2-c]pyridin-5-yl)-pentyl]-3-ethyl-1,3-dihydro-2*H*-indol-2-on,
3-[5-(2-Chlor-6,7-dihydro-4*H*-thieno[3,2-c]pyridin-5-yl)-pentyl]-3-ethyl-1,3-dihydro-2*H-*indol-2-on,
3-[4-(3,4-dihydro-1*H*-isochinolin-2-yl)-butyl]-3-ethyl-1,3-dihydro-2*H*-indol-2-on,
3-Ethyl-3- {4-[4-(4-fluorphenyl)-1,2,3,6-tetrahydropyridin-1-yl]-butyl}-1,3-dihydro-2*H-*indol-2-on,
3-Ethyl-3-{4-[4-(4-fluorphenyl)-1,2,3,6-tetrahydropyridin-1-yl]-butyl}-1,3-dihydro-2*H-*indol-2-on,
3-{4-[4-(4-Chlorphenyl)-3,6-dihydro-2*H*-pyridin-1-yl]-butyl}-3-ethyl-1,3-dihydro-2*H-*indol-2-on,
3-{4-[4-(3-Chlorphenyl)-3,6-dihydro-2*H*-pyridin-1-yl]-butyl}-3-ethyl-1,3-dihydro-2*H-*indol-2-on,
und pharmazeutisch annehmbare Säureadditionssalze davon.

4. 3-[5-(6,7-Dihydro-4*H*-thieno[3,2-c]pyridin-5-yl)-pentyl]-3-ethyl-1,3-dihydro-2*H*-indol-2-on gemäß Anspruch 1 und pharmazeutisch annehmbare Säureadditionssalze davon.

5. 3-Ethyl-3- {4-[4-(4-fluorphenyl)-1,2,3,6-tetrahydropyridin-1-yl]butyl}-1,3-dihydro-2*H-*indol-2-on gemäß Anspruch 1 und pharmazeutisch annehmbare Säureadditionssalze davon.

6. Pharmazeutische Zusammensetzungen, umfassend als aktiven Bestandteil mindestens eine der Verbindungen der allgemeinen Formel (I) oder ein pharmazeutisch annehmbares Säureadditionssalz davon in Vermischung mit einem oder mehreren herkömmlichen Träger(n) oder Hilfsstoff(en).

7. Pharmazeutische Zusammensetzungen gemäß Anspruch 6, die zur Behandlung oder Prophylaxe von Störungen des zentralen Nervensystems, insbesondere Depression, Angst, Schizophrenie, Stimmungsstörungen, Manie, mentalem Verfall, Schlaganfall, Zelltod in bestimmten Bereichen des zentralen Nervensystems, Stresserkrankung, gastrointestinalen Erkrankungen oder kardiovaskulären Erkrankungen brauchbar sind.

8. Pharmazeutische Zusammensetzungen gemäß Anspruch 6 oder 7, umfassend als aktiven Bestandteil 3-[5-(6,7-Dihydro-4*H*-thieno[3,2-c]pyridin-5-yl)-pentyl]-3-ethyl-1,3-dihydro-2*H*-indol-2-on oder 3-Ethyl-3-{4-[4-(4-fluorphenyl)-1,2,3,6-tetrahydropyridin-1-yl]-butyl}-1,3-dihydro-2*H*-indol-2-on oder pharmazeutisch annehmbare Säureadditionssalze davon in Vermischung mit einem oder mehreren herkömmlichen Träger(n) oder Hilfsstoff(en).

9. Verfahren zur Herstellung einer Verbindung der allgemeinen Formel (I), worin
R¹ und R² unabhängig Wasserstoff, Halogen, Alkyl mit 1 bis 7 Kohlenstoffatom(en) oder Sulfamoyl bedeuten;
R³ Wasserstoff oder gerad- oder verzweigtkettiges Alkyl mit 1 bis 7 Kohlenstoffatom(en) bedeuten;
R⁴ für Alkyl mit 1 bis 7 Kohlenstoffatom(en) steht;
R⁵ Wasserstoff ist und R⁶ Phenyl bedeutet, das gegebenenfalls 1 bis 3 Substituent(en) trägt, die aus Halogen und Alkyl mit 1 bis 7 Kohlenstoffatom(en), 1 bis 3 Halogensubstituent(en) tragend, gewählt wird, oder
R⁵ und R⁶ zusammen mit den benachbarten Kohlenstoffatomen des Tetrahydropyridinrings eine Phenylgruppe oder einen 5- oder 6-gliedrigen heterocyclischen Ring, der ein Schwefelatom als Heteroatom enthält, bilden können, welcher gegebenenfalls einen Halogensubstituenten tragen kann;
m 1, 2, 3, 4, 5 oder 6 ist,
welches folgendes umfasst:
(a) die Umsetzung einer Verbindung der allgemeinen Formel (II), 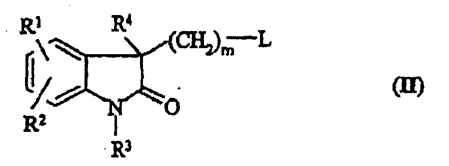 worin R¹-R⁴ wie oben angegeben sind und L eine Abgangsgruppe, vorzugsweise Chlor oder Brom, ist, m 1, 2, 3, 4, 5 oder 6 ist, mit einem Piperidinderivat der allgemeinen Formel (III) 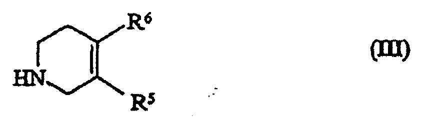 worin R⁵ und R⁶ wie oben angegeben sind, oder
(b) die Umsetzung einer Verbindung der allgemeinen Formel (IV) 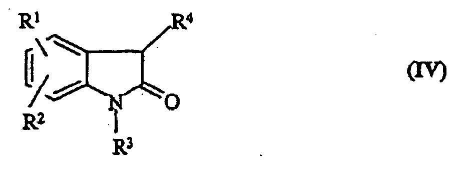 worin R¹, R², R³ und R⁴ wie oben angegeben sind, mit einer Verbindung der allgemeinen Formel (V)
L-(CH₂)ₘ-L' (V)
worin m 1, 2, 3, 4, 5 oder 6 ist, L und L' eine Abgangsgruppe, vorzugsweise Chlor oder Brom, bedeuten, in Gegenwart einer starken Base, gegebenenfalls das Halogenieren der so erhaltenen Verbindung der allgemeinen Formel (II), worin R² Wasserstoff ist, und das Umsetzen der so erhaltenen Verbindung der allgemeinen Formel (II), worin L eine Abgangsgruppe, vorzugsweise Chlor oder Brom, ist, R² Wasserstoff oder Halogen ist und m 1, 2, 3, 4, 5 oder 6 ist, mit einem Pyridinderivat der allgemeinen Formel (III), worin R⁵ und R⁶ wie oben angegeben sind, in Gegenwart eines Säurebindungsmittels, oder
(c) das Umsetzen einer Verbindung der allgemeinen Formel (IV), worin R¹, R², R³ und R⁴ wie oben angegeben sind, mit einem Pyridinderivat der allgemeinen Formel (VI) 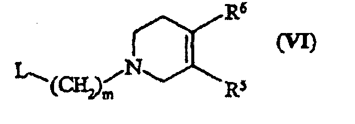 worin L Sulfonyloxy oder Halogen, vorzugsweise Chlor oder Brom, ist; R⁵ und R⁶ wie oben angegeben sind; m 1, 2, 3, 4, 5 oder 6 ist, in Gegenwart einer starken Base und gegebenenfalls das Halogenieren des so erhaltenen Produktes, wobei R² Wasserstoff ist, oder das Freisetzen der freien Base aus einem Salz davon oder das Umwandeln desselben in ein pharmazeutisch annehmbares, organisches oder anorganisches Säureadditionssalz davon.

10. Verbindungen der allgemeinen Formel (I) gemäß einem beliebigen der Ansprüche 1 - 5 zur Verwendung als ein Medikament.

11. Verfahren zur Herstellung von pharmazeutischen Zusammensetzungen, die für die Behandlung oder Prophylaxe von Störungen des zentralen Nervensystems, insbesondere Depression, Angst, Schizophrenie, Stimmungsstörungen, Manie, mentalem Verfall, Schlaganfall, Zelltod in bestimmten Bereichen des zentralen Nervensystems, Stresserkrankung, gastrointestinalen Erkrankungen oder kardiovaskulären Erkrankungen, brauchbar sind, welches das Vermischen von mindestens einer Verbindung der allgemeinen Formel (I) oder eines pharmazeutisch annehmbaren Säureadditionssalzes davon gemäß mindestens einem der Ansprüche 1 bis 5 oder pharmazeutisch annehmbaren Säureadditionssalzen davon mit einem pharmazeutischen Träger und gegebenenfalls einem anderen Hilfsmitteln und das Überführen der Mischung zu einer galenischen Form umfasst.

12. Verwendung einer pharmazeutischen Zusammensetzung, die mindestens eine Verbindung der allgemeinen Formel (I) oder ein pharmazeutisch annehmbares, organisches oder anorganisches Säureadditionssalz davon enthält, bei der Herstellung eines Medikamentes zur Behandlung oder Prophylaxe von Störungen des zentralen Nervensystems, insbesondere Depression, Angst, Schizophrenie, Stimmungsstörung, Manie, mentalem Verfall, Herzschlag, Zelltod in bestimmten Bereichen des zentralen Nervensystems, Stresserkrankung, gastrointestinalen Erkrankungen, kardiovaskulären Erkrankungen.

13. Verfahren gemäß den Varianten (a) - (c) vom Anspruch 9, welches die Durchführung der Reaktion in einem apolaren, dipolar aprotischen oder polar-protischen Lösungsmittel, wie einem aliphatischen Kohlenwasserstoff oder halogenierten aliphatischen Kohlenwasserstoff, oder in einem Lösungsmittel vom aromatischem Kohlenwasserstoff-, Ether-, Ester-, Nitril- oder Keton-Typ oder in einem gerad- oder verzweigtkettigen Alkohol mit 1 bis 4 Kohlenstoffatom(en)oder in Mischungen von solchen Lösungsmitteln umfasst.

14. Verfahren gemäß mindestens einem der Varianten (a) - (c) von Anspruch 9, welches die Durchführung der Reaktion in der Schmelze umfasst.

## Revendications

1. Les dérivés du 3,3-Disubstitués indol-2-one de Formule générale (I), 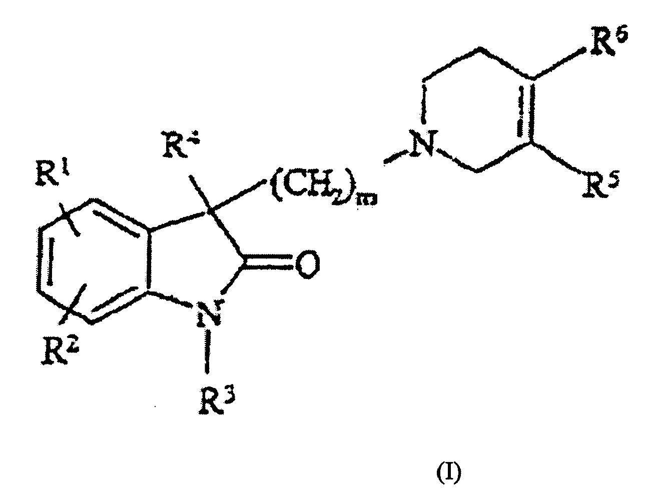 où
R¹ et R² représentent indépendamment un hydrogène, un halogène, un alkyle ayant de 1 à 7 atome(s) de carbone ou de sulfamoyle;
R³ représente un hydrogène ou un alkyle à chaîne droite ou ramifiée ayant de 1 à 7 atome(s) de carbone;
R⁴ représente un alkyle ayant de 1 à 7 atome(s) de carbone;
R⁵ est un hydrogène et R⁶ représente un phényle porteur le cas échéant de 1 à 3 substituant(s) choisis parmi un halogène et un alkyle ayant de 1 à 7 atome(s) de carbone porteur(s) de 1 à 3 substituant(s) halogène, ou
R⁵ et R⁶ forment, conjointement avec les atomes de carbone adjacents du cycle de tétrahydropyridine, un groupe de phényle ou un cycle hétérocyclique à 5 ou 6 membres comportant un sulfure comme hétéroatome porteur le cas échéant d'un substituant halogène;
m représente 1, 2, 3, 4, 5 ou 6,
et des sels d'addition acides pharmaceutiquement acceptables issus de ces composés.

2. Les dérivés du 3,3-Disubstitués indol-2-one de Formule générale (I), selon la revendication 1 où
R¹, R², et R³ représentent indépendamment un hydrogène, un halogène ou un alkyle à chaîne droite ou ramifiée ayant de 1 à 7 atome(s) de carbone, R⁴ est un éthyle, R⁵ représente un hydrogène et R⁶ représente un phényle, porteur le cas échéant d'un substituant halogène ou trifluorométhyle, ou
R⁵ et R⁶ forment, conjointement avec les atomes de carbone adjacents du cycle de tétrahydropyridine, un phényle ou un cycle hétérocyclique de 5 ou 6 membres comportant un atome de sulfure comme hétéroatome, porteur le cas échéant d'un atome halogène, m représente 3, 4 ou 5, et des sels d'addition acides pharmaceutiquement acceptables issus de ces composés.

3. Les composés de Formule générale (I) selon la revendication 1 :
3-éthyle-3-{4-[4-(3-trifluorométhyle-phényle)-1,2,3,6-tétrahydropyridine-1-yl]-butyl}-1,3-dihydro-2*H*-indol-2-one,
3-[4-(6,7-dihydro-4*H*-thiéno[3,2-c]pyridine-5-yl)-butyl]-3-éthyl-1,3-dihydro-2*H-*indol-2-one,
3-[5-(6,7-dihydro-4*H*-thiéno[3,2-c]pyridine-5-yl)-pentyl]-3-éthyl-1,3-dihydro-2*H-*indol-2-one,
3-[5-(2-chloro-6,7-dihydro-4*H*-thiéno[3,2-c]pyridine-5-yl)pentyl]-3-éthyl-1,3-dihydro-2*H*-indol-2-one,
3-[4-(3,4-dihydro-1*H*-isoquinoline-2-yl)-butyl]-3-éthyl-1,3-dihydro-2*H*-indol-2-one,
3-éthyl-3-{4-[4-(4-fluorophényl)-1,2,3,6-tétrahydropyridine-1-yl]-butyl}-1,3-dihydro-2*H*-indol-2-one,
3-éthyl-3- {4-[4-(4-fluorophényl)-1,2,3,6-tétrahydropyridine-1-yl]-butyl}-1,3-dihydro-2*H*-indol-2-one,
3- {4-[4-(4-chlorophényl)-3,6-dihydro-2*H*-pyridine-1-yl]-butyl}-3-éthyl-1,3-dihydro-2*H*-indol-2-one,
3-{4-[4-(3-chlorophényl)-3,6-dihydro-2*H*-pyridine-1-yl]-butyl}-3-éthyl-1,3-dihydro-2*H*-indol-2-one,
et des sels d'addition acides pharmaceutiquement acceptables issus de ces composés.

4. 3-[5-(6,7-Dihydro-4*H*-thiéno[3,2-c]pyridine-5-yl)-pentyl]-3-éthyl-1,3-dihydro-2*H-*indol-2-one selon la revendication 1 et des sels d'addition acides pharmaceutiquement acceptables issus de ces composés.

5. 3-Éthyl-3- {4-[4-(4-fluorophényl)-1,2,3,6-tétrahydropyridine-1-yl]-butyl}-1,3-dihydro-2*H*-indol-2-one selon la revendication 1, et des sels d'addition acides pharmaceutiquement acceptables issus de ces composés.

6. Les compositions pharmaceutiques comportant comme ingrédient actif au moins un des composés de Formule générale (I) ou un sel d'addition acide pharmaceutiquement acceptable issu de ces composés en mélange avec un ou plusieurs support(s) habituel(s) ou agent(s) auxiliaire(s).

7. Les compositions pharmaceutiques selon la revendication 6 sont utiles pour le traitement ou la prophylaxie des troubles du système nerveux central, en particulier la dépression, l'anxiété, la schizophrénie, les troubles de l'humeur, les manies, le déclin mental, les attaques, la mort de cellule de certaines zones du système nerveux central, les maladies liées au stress, des maladies gastro-intestinales et des maladies cardiovasculaires.

8. Les compositions pharmaceutiques selon la revendication 6 ou 7 comportant comme ingrédient actif 3-[5-(6,7-dihydro-4*H*-thiéno[3,2-c]pyridine-5-yl)-pentyl]-3-éthyl-1,3-dihydro-2*H*-indol-2-one ou 3-éthyl-3-{4-[4-(4-fluorophényl)-1,2,3,6-tétrahydropyridine-1-yl]-butyl}-1,3-dihydro-2*H*-indol-2-one ou des sels d'addition acides pharmaceutiquement acceptables issus de ces composés en mélange avec un ou plusieurs support(s) habituel(s) ou agent(s) auxiliaire(s).

9. Procédé de préparation d'un composé de Formule générale (I), où
R¹ et R² représentent indépendamment un hydrogène, un halogène, un alkyle ayant de 1 à 7 atome(s) de carbone ou de sulfamoyle;
R³ représente un hydrogène ou un alkyle à chaîne droite ou ramifiée ayant de 1 à 7 atome(s) de carbone;
R⁴ représente un alkyle ayant de 1 à 7 atome(s) de carbone;
R⁵ est un hydrogène et R⁶ représente un phényle porteur le cas échéant de 1 à 3 substituant(s) choisis parmi un halogène et un alkyle ayant de 1 à 7 atome(s) de carbone porteur(s) de 1 à 3 substituant(s) halogène, ou
R⁵ et R⁶ forment, conjointement avec les atomes de carbone adjacents du cycle de tétrahydropyridine, un groupe de phényle ou un cycle hétérocyclique de 5 ou 6 membres comportant un sulfure comme hétéroatome porteur le cas échéant d'un substituant halogène;
m représente 1, 2, 3, 4, 5 ou 6,
qui consiste
(a) à faire réagir un composé de Formule générale (II), 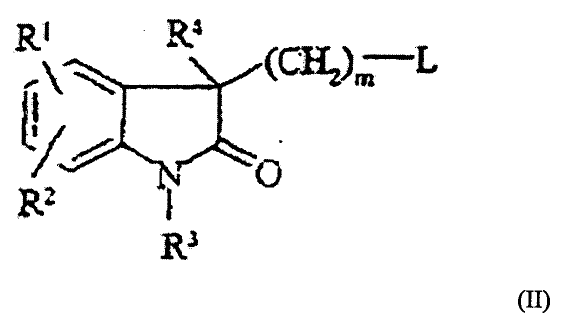 où R¹-R⁴ sont tels que décrits ci-dessus et L est un groupe partant, de préférence de chlore ou de brome, m représente 1, 2, 3, 4, 5 ou 6, avec un dérivé de pipéridine de Formule générale (III) 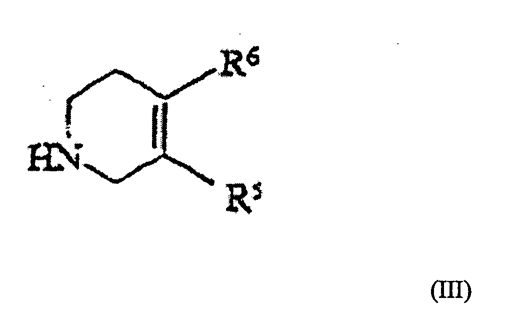 où R⁵ et R⁶ sont tels que décrits ci-dessus, ou
(b) à faire réagir un composé de Formule générale (IV) 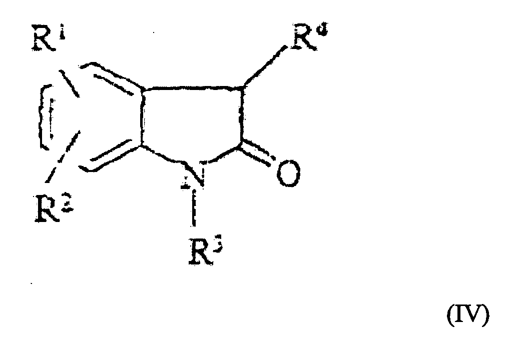 où R¹, R², R³ et R⁴ sont tels que décrits ci-dessus, avec un composé de Formule générale (V),
L-(CH₂)ₘ-L' (V)
où m représente 1, 2, 3, 4, 5 ou 6, L et L' représentent un groupe partant, de préférence de chlore ou de brome, en présence d'une base forte, 1e cas échéant par halogénation du composé ainsi obtenu de Formule générale (II), où R² est un hydrogène et 1a réaction du composé ainsi obtenu de Formule générale (II), où L est un groupe partant, de préférence de chlore ou de brome, R² est un hydrogène ou un halogène et m représente 1, 2, 3, 4, 5 ou 6, avec un dérivé de pyridine de Formule générale (III) où R⁵ et R⁶ sont tels que décrits ci-dessus, en présence d'un agent de fixation aux acides, ou
(c) à faire réagir un composé de Formule générale (IV), où R¹, R², R³, et R⁴ sont tels que décrits ci-dessus, avec un dérivé de pyridine de Formule générale (VI), 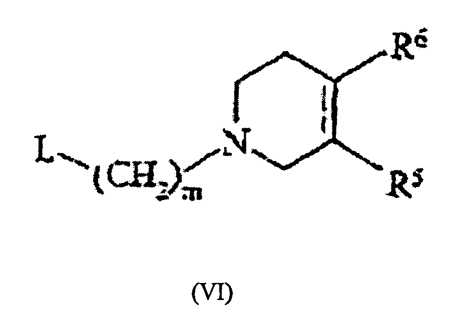 où L est un sulfonyloxy ou un halogène, de préférence de chlore ou de brome; R⁵ et R⁶ sont tels que décrits ci-dessus; m représente 1, 2, 3, 4, 5 ou 6, en présence d'une base forte et le cas échéant d'une halogénation du produit ainsi obtenu, où R² est un hydrogène, ou en libérant la base libre d'un de ses sels ou en le transformant en sel d'addition acide pharmaceutiquement acceptable, organique ou non, issu de ces composés.

10. Des composés de Formule générale (I) selon l'une des revendications 1 - 5 pour l'utilisation d'un médicament.

11. Procédé pour 1a préparation de compositions pharmaceutiques utiles pour 1e traitement ou la prophylaxie des troubles du système nerveux central, en particulier la dépression, l'anxiété, la schizophrénie, les troubles de l'humeur, les manies, le déclin mental, les attaques, la mort de cellule de certaines zones du système nerveux central, des maladies liées au stress, des maladies gastro-intestinales ou des maladies cardiovasculaires, comprenant le mélange d'au moins un composé de Formule générale (I) ou de sels d'addition acides pharmaceutiquement acceptables issus de ces composés selon l'une des revendications 1 à 5 ou des sels d'addition acides pharmaceutiquement acceptables issus de ces composés avec un excipient pharmaceutique et le cas échéant d'autre agent auxiliaire et en présentant le mélange sous forme galénique.

12. Utilisation d'une composition pharmaceutique contenant au moins un composé de Formule générale (I) ou de sel d'addition acide pharmaceutiquement acceptable, organique ou non, issu de ces composés pour la préparation d'un médicament pour le traitement ou la prophylaxie des troubles du système nerveux central, en particulier la dépression, l'anxiété, la schizophrénie, les troubles de l'humeur, les manies, le déclin mental, les attaques, la mort de cellule de certaines zones du système nerveux central, des maladies liées au stress, des maladies gastro-intestinales ou des maladies cardiovasculaires.

13. Procédé selon des variantes (a) - (c) de la revendication 9, qui consiste à effectuer la réaction dans un solvant apolaire, dipolaire, aprotique ou un solvant protique polaire, tel qu'un hydrocarbone aliphatique ou un hydrocarbone aliphatique halogéné, ou dans un solvant d'hydrocarbone aromatique, de type éther, ester, nitrile ou cétone, ou d'alcool à chaîne droite ou ramifiée ayant de 1 à 4 atome(s) de carbone ou des mélanges de tels solvants.

14. Procédé selon l'une des variantes (a) - (c) de la revendication 9, qui consiste à effectuer la réaction dans le mélange.
